# EUROPEAN PATENT SPECIFICATION

(11) **EP 0 888 130 B1**
(45) Date of publication and mention of the grant of the patent: **02.07.2003**
(21) Application number: 97915928.2
(22) Date of filing: 12.03.1997
(51) Int. Cl.: A61K 51/08

(54) **NEW TERNARY RADIOPHARMACEUTICAL COMPLEXES**
TERNÄRE RADIOPHARMAZEUTISCHE KOMPLEXE
COMPLEXES TERNAIRES RADIO-PHARMACEUTIQUES

(30) Priority: 13.03.1996 US 613360; 08.05.1996 US 646866; 28.02.1997 US 808699
(43) Date of publication of application: 07.01.1999
(73) Proprietor: Bristol-Myers Squibb Pharma Company, Princeton, New Jersey 08443-4000 (US)
(72) Inventor: EDWARDS, David, Scott, Burlington, MA 01803 (US); LIU, Shuang, Chelmsford, MA 01824 (US)
(74) Representative: Carpmaels & Ransford
(86) International application number: US9703915
(87) International publication number: WO97033627

(56) References cited:
- WO-A-92/05154
- WO-A-93/15771
- WO-A-94/16741
- WO-A-94/22494
- WO-A-95/03280
- WO-A-96/31243
- WO-A-96/40637
- US-A- 5 350 837
- HARRIS ET AL.: "Design and synthesis of Tc-99m labeled boroarginine thrombin inhibitors as potential thrombus imaging agents" J. NUCL. MED., vol. 35, no. sup5, May 1994, pages 241p-242p, XP002050941
- LIU ET AL.: "Labeling a hydrazino nicotinamide-modified cyclic IIb/IIIa receptor antagonist with 99mTc using aminocarboxylates as coligands" BIOCONJUGATE CHEM., vol. 7, no. 1, January 1996 - February 1996, pages 63-71, XP002050942
- EDWARDS: "Potential 99mTc radiopharmaceuticals for renal immaging : Tris(N-substituted-3-hydroxy-2-methyl-4-py ridinato)technetium(IV) cations" NUCL. MED. BIOL., vol. 20, no. 7, October 1903, pages 857-863, XP002050943
- RAJOPADHYE : "Synthesis, evaluation and Tc-99m complexation of a hydrazinonicotinyl conjugate of a GP IIb/IIIa antagonist cyclic peptide for the detection of deep vein thrombosis" BIOORG. MED. CHEM. LETT., vol. 7, no. 8, 1997, pages 955-960, XP002050944

## Description

This invention relates to novel radiopharmaceuticals which are useful as imaging agents for the diagnosis of cardiovascular disorders, infectious disease and cancer, and to kits useful for their preparation. The radiopharmaceuticals are comprised of nitrogen-containing heterocycle ligated technetium-99m labeled hydrazino or diazino modified biologically active molecules that selectively localize at sites of disease and thus allow an image to be obtained of the loci using gamma scintigraphy.

### BACKGROUND

There is a current need for new methods for the non-invasive diagnosis of a variety of diseases such as thromboembolic disease, atherosclerosis, infection and cancer. Radiopharmaceuticals comprised of gamma-ray emitting radionuclide labeled biologically active molecules can fulfill the need. The biologically active molecules serve to localize the radionuclides at the sites of disease and thus allow the sites to be visualized by gamma scintigraphy. The molecules can be either proteins, antibodies, antibody fragments, peptides or polypeptides, or peptidomimetics. The molecules interact with a receptor or binding site expressed at the sites of the disease or with a receptor or binding site on an endogenous blood component, such as platelets and leukocytes, that accumulate at the sites. This interaction results in selective localization of a percentage of the injected radiopharmaceutical while the remainder is cleared either through the renal or hepatobiliary systems. The localized radiopharmaceutical is then imaged externally using gamma scintigraphy. The relative rates of sequestration, clearance and radionuclidic decay determine the ease of visualization, often expressed as the target-to-background ratio. Frequently, only certain portions of the biologically active molecules bind to the receptors; these portions are termed the recognition sequences or units.

A number of radiopharmaceuticals comprised of radionuclide labeled proteins, antibodies or antibody fragments are under development, however, to date only one has been approved by the Food and Drug Administration. This sparse record results from a combination of factors that make developing these radiopharmaceuticals difficult, including problems with manufacturing and quality control, non-optimal sequestration and clearance rates, and the occurence of antigenic or allergic responses to the radiopharmaceuticals. These problems are mainly due to the macromolecular nature of the proteins, antibodies and antibody fragments. Their high molecular weight makes direct chemical synthesis impractical, therefore they must be synthesized by recombinant or cloning techniques that typically give low yields and require extensive isolation and purification procedures. Their molecular weight can slow their rates of localization and preclude their clearance by an active elimination mechanism via the kidneys or liver, resulting in prolonged retention in the circulation which causes a high background level during imaging. Also, the body's immune system tends to recognize more efficiently larger exogenous species.

The use of lower molecular weight peptides, polypeptides or peptidomimetics as the biologically active molecules obviates a number of these problems. These molecules can be synthesized directly using classical solution chemistry or by an automated peptide synthesizer. They can be formed in higher yields and require less complicated purification procedures. They tend to clear more rapidly from the circulation by an active elimination pathway resulting in a lower background in the images. They are also usually not immunogenic. The first radionuclide labeled polypeptide radiopharmaceutical has been recently approved by the Food and Drug Administration.

There are two general methods for labeling biologically active molecules with radionuclides for use as radiopharmaceuticals termed direct and indirect labeling. Direct labeling involves attaching the radionuclide to atoms on the biologically active molecule; while the indirect method involves attaching the radionuclide via a chelator. The chelator can either be attached to the biologically active molecule prior to reaction with the radionuclide or the radionuclide labeled chelator moiety can be attached to the biologically active molecule. Several recent reviews describe these labeling methods and are incorporated herein by reference: S. Jurisson et. al., Chem. Rev., 1993, 93, 1137; A. Verbruggen, Eur. J. Nuc. Med., 1990, 17, 346; and M. Derwanjee, Semin. Nuc. Med., 1990, 20, 5.

The use of hydrazines and hydrazides as chelators to modify proteins for labeling with radionuclides has been recently disclosed in Schwartz et. al., U.S. Patent 5,206,370. For labeling with technetium-99m, the hydrazino-modified protein is reacted with a reduced technetium species, formed by reacting pertechnetate with a reducing agent in the presence of a chelating dioxygen ligand. The technetium becomes bound to the protein through what are believed to be hydrazido or diazenido linkages with the coordination sphere completed by the ancillary dioxygen ligands. Examples of ancillary dioxygen ligands include glucoheptonate, gluconate, 2-hydroxyisobutyrate, and lactate.

Certain dioxygen ligands have been recently reported to be particularly advantageous for labeling hydrazino-modified proteins with technetium-99m. Bridger et. al., U. S. Patent 5,350,837, disclose a series of functionalized aminocarboxylates the use of which are reported to improve the labeling process of hydrazino-modified macromolecules such as monoclonal antibodies. The improvements are manifest by shorter reaction times and higher specific activities. Examples of these improved dioxygen ligands include hydroxyalkyl substituted glycine derivatives such as tricine.

In co-pending U.S. Ser. No. 08/218,861 (equivalent to WO 94/22494) , filed March 28, 1994, the synthesis of novel radiolabeled platelet IIb/IIIa receptor antagonists as imaging agents for thromboembolic disorders is disclosed. These reagents comprise radionuclide labeled chelator modified cyclic compounds. A preferred chelator for modifying the cyclic compounds is the hydrazino or diazenido moiety. The preferred reagents are used to synthesize binary complexes comprised of the hydrazido or diazenido moiety and one of a series of ancillary ligands.

### SUMMARY OF THE INVENTION

This invention provides novel radiopharmaceuticals which are useful as imaging agents for the diagnosis of cardiovascular disorders, such as thromboembolic disease or atherosclerosis, infectious disease and cancer. The radiopharmaceuticals are comprised of nitrogen-containing heterocycle ligated technetium-99m labeled hydrazino or diazenido modified biologically active molecules that selectively localize at sites of disease and thus allow an image to be obtained of the loci using gamma scintigraphy. The invention also provides methods of using said radiopharmaceuticals as imaging agents for the diagnosis of cardiovascular disorders, such as thromboembolic disease or atherosclerosis, infectious disease and cancer. It further provides kits for the preparation of said radiopharmaceuticals.

### Brief Description of the Figures

Figure 1. Data from the Canine Arteriovenous Shunt model for the Radiopharmaceutical of Example 1 (150 µCi/kg,i.v.), compared to ¹¹¹In-platelets, ¹²⁵I-fibrinogen and ^{99m}Tc-albumin.

### DETAILED DESCRIPTION OF THE INVENTION

The present invention is directed to novel radiopharmaceuticals for the diagnosis of cardiovascular disorders, such as thromboembolic disease and atherosclerosis, infectious disease or cancer of the formula [(Q)_{d'}Lₙ-C_{h'}]ₓ-Mₜ(A_{L1})_{y}(A_{L2})₂, methods of using said radiopharmaceuticals in the diagnosis of diseases and kits useful for the preparation of said radiopharmaceutical.

The present invention provides a radiopharmaceutical of formula:

**[(Q)**_{**d'**}**L**_{**n**}**-C**_{**h'**}**]**_{**x**}**-M**_{**t**}**(A**_{**L1**}**)**_{**y**}**(A**_{**L2**}**)**_{**z**} (1)

and pharmaceutically acceptable salts thereof wherein,
Q is a biologically active molecule selected from the group: IIb/IIIa receptor antagonists. IIb/IIIa receptor ligands, fibrin binding peptides, leukocyte binding peptides, chemotactic peptides, somatostatin analogs, and selectin binding peptides;
d' is 1 to 3;
Lₙ is:

   -(CR⁵⁵R⁵⁶)_{g"}-[Y¹(CR⁵⁵R⁵⁶)_{f}Y²]_{f'}-(CR⁵⁵R⁵⁶)_{g"}-,

   wherein:
   g" is 0-5;
   f is 0-5;
   f' is 1-5;
   Y¹ and Y², at each occurrence, are independently selected from: O, NR⁵⁶, C=O, C(=O)O, OC(=O)O, C(=O)NH, C=NR⁵⁶, S, SO, SO₂, SO₃, NHC(=O), (NH)₂C(=O), (NH)₂C=S;
   R⁵⁵ and R⁵⁶ are independently selected at each occurrence from:
      hydrogen, C₁-C₁₀ alkyl and alkaryl;
x and y are 1;
Z is 1 or 2;
Mₜ is ^{99m}Tc;
C_{h'} is selected from the group: R⁴⁰N=N⁺= and R⁴⁰R⁴¹N-N=; wherein
   R⁴⁰ is independently selected at each occurrence from the group: aryl substituted with 0-3 R⁵², and heterocycle substituted with 0-3 R⁵²;
   R⁴¹ is independently selected from the group: hydrogen, aryl substituted with 0-1 R⁵², C₁-C₃ alkyl substituted with 0-1 R⁵², and a heterocycle substituted with 0-1 R⁵²;
   R⁵² is independently selected at each occurrence from the group: a bond to Lₙ, -CO₂R⁵³, -CH₂OR⁵³, -SO₃H, -SO₂R^{53a}, -N(R⁵³)₂, -N(R⁵³)_{3⁺} -NHC(=NH)NHR⁵³, and -OCH₂CO₂H;
   R⁵³ and R^{53a} are each independently selected at each occurrence from the group: hydrogen and C₁-C₃ alkyl;
A_{L1} is a functionalized aminocarboxylate;
A_{L2} is an ancillary ligand capable of stabilizing the radiopharmaceutical selected from the group: 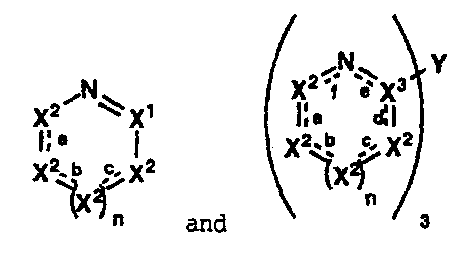 wherein
   n is 0
   X¹ is independently selected at each occurrence from the group: CR⁶⁴ and N;
   X² is independently selected at each occurrence from the group: CR⁶⁴, CR⁶⁴R⁶⁴, N, NR⁶⁴, 0 and S;
   X³ is independently selected at each occurrence from the group: C, CR⁶⁴, and N;
   provided the total number of heteroatoms in each ring of the ligand A_{L2} is 1 to 4;
   Y is BR⁶⁴⁻;
   and a, b, c, d, e and f indicate the positions of optional double bonds, provided that one of e and f is a double bond.
   R⁶⁴ is independently selected at each occurrence from the group:
      H, C₁-C₃ alkyl substituted with 0-3 R⁶⁵, aryl substituted with 0-3 R⁶⁵, and R⁶⁵;
   or, alternatively, two R⁶⁴ may be taken together with the atom or atoms to which they are attached to form a fused aromatic or heterocyclic ring, substituted with 0-3 R⁶⁵;
   R⁶⁵ is independently selected at each occurrence from the group: -NO₂, -CO₂R⁶⁶, -OR⁶⁶, -SO₃H, and -OCH₂CO₂H; and
   R⁶⁶ is hydrogen.

Preferred radiopharmaceuticals of the present are wherein:
Q is a biologically active molecule selected from the group: IIb/IIIa receptor antagonists and chemotactic peptides;
d' is 1;
   Y¹ and Y², at each occurrence, are independently selected from:
   O, NR⁵⁶, C=O, C(=O)O, OC(=O)O, C(=O)NH, C=NR⁵⁶, NHC(=O), (NH)₂C(=O);
   R⁵⁵ and R⁵⁶ are hydrogen;
z is 1;
   R⁴⁰ is heterocycle substituted with R⁵²;
   R⁴¹ is hydrogen;
   R⁵² is a bond to Lₙ;
   A_{L1} is tricine;
      X² is independently selected at each occurrence from the group: CR⁶⁴, CR⁶⁴R⁶⁴, N, NR⁶⁴ and O;
      X³ is N;
      provided the total number of heteroatoms in each ring of the ligand A_{L2} is 2 to 3;
      R⁶⁴ is independently selected at each occurrence from the group:
         H, C₁-C₃ alkyl substituted with 0-1 R⁶⁵, aryl substituted with 0-1 R65; and R₆₅;
      R⁶⁵ is independently selected at each occurrence from the group: -NO₂, -CO₂R⁶⁶, -OR⁶⁶, and -SO₃H.

More preferably, the present invention provides a radiopharmaceutical wherein:
Q is 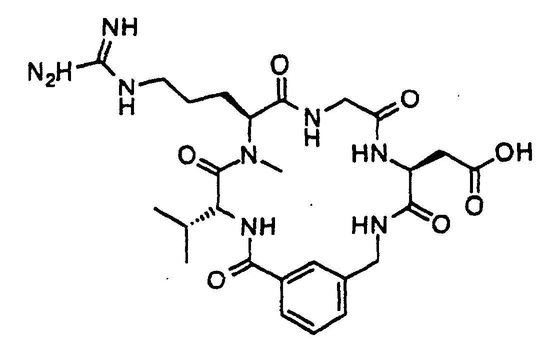
d' is 1;
Lₙ is attached to Q at the carbon atom designated with a * and has the formula:

   -(C=O)NH(CH₂)₅C(=O)NH-;
C_{h'} is 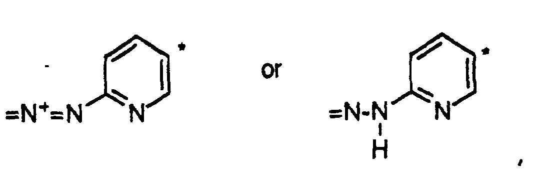 and is attached to Ln at the carbon atom designated with a *;
Mₜ iS ^{99m}Tc;
A_{L1} is tricine;
x, y and z are l;
and A_{L2} is selected from the group:

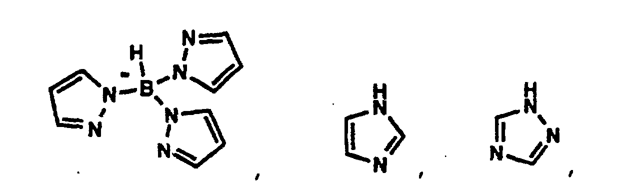

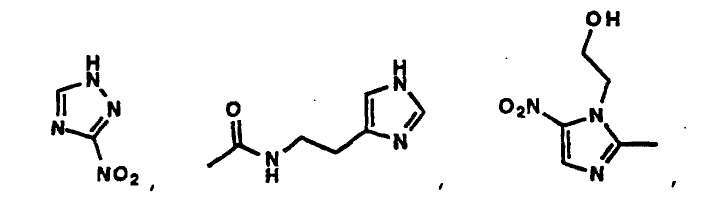

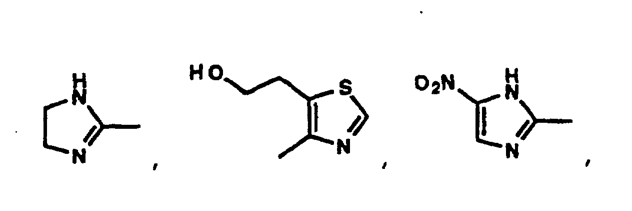

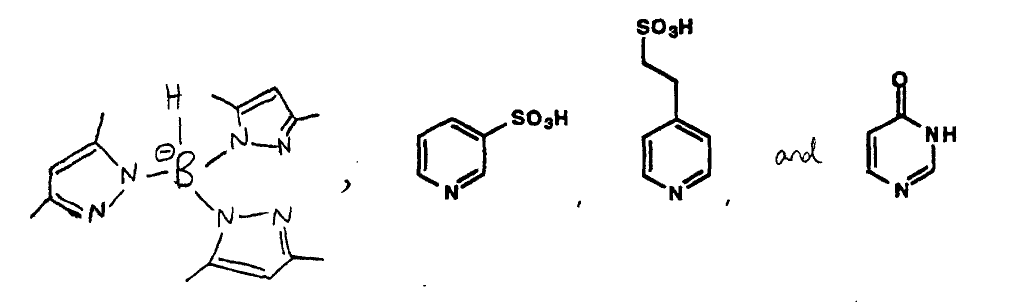

Another embodiment of this invention is the use of a radiopharmaceutical, as defined hereinabove, for the preparation of a medicament for use in radioimaging a mammal.

Another embodiment of this invention is the use of a radiopharmaceutical, as defined hereinabove, for the preparation of a medicament for use in visualizing sites of platelet deposition in a mammal by radioimaging.

Another embodiment of this invention is the use of a radiopharmaceutical, as defined hereinabove, for the preparation of a medicament for use in determining platelet deposition in a mammal.

Another embodiment of this invention is the use of a radiopharmaceutical, as defined hereinabove, for the preparation of a medicament for use in diagnosing a disorder associated with platelet deposition in a mammal.

The present invention further provides a kit for preparing a radiopharmaceutical comprising:
(a) a predetermined quantity of a sterile, pharmaceutically acceptable reagent of formula:

   **(Q)**_{**d'**}**L**_{**n**}**C**_{**h**}**;**
(b) a predetermined quantity of a sterile, pharmaceutically acceptable first ancillary ligand, A_{L1}, which is a functionalized aminocarboxylate;
(c) a predetermined quantity of a sterile, pharmaceutically acceptable second ancillary ligand, A_{L2}, selected from the group: 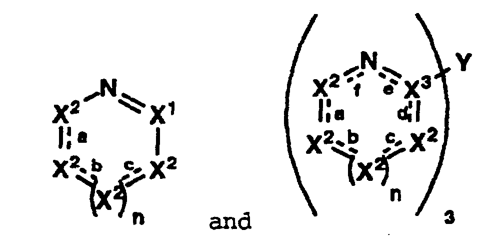
(d) a predetermined quantity of a sterile, pharmaceutically acceptable reducing agent; and
(e) optionally, a predetermined quantity of one or more sterile, pharmaceutically acceptable components selected from the group: transfer ligands, buffers, lyophilization aids, stabilization aids, solubilization aids and bacteriostats;
wherein:
Q, d', Lₙ, X¹, X², X³, n, Y, a, b, c, d, e and f are as defined hereinabove.
Cₕ is a radionuclide metal chelator selected from the group: R⁴⁰R⁴¹N-N=C(C₁-C₃ alkyl)₂ and R⁴⁰NNH₂-, and R⁴⁰R⁴¹N-N=CR⁸⁰R⁸¹, wherein,
   R⁴⁰ is independently selected at each occurrence from the group: a bond to Lₙ, C₁-C₁₀ alkyl substituted with 0-3 R⁵², aryl substituted with 0-3 R⁵², cycloaklyl substituted with 0-3 R⁵², heterocycle substituted with 0-3 R^{52,} heterocycloalkyl substituted with 0-3 R⁵², aralkyl substituted with 0-3 R⁵² and alkaryl substituted with 0-3 R52;
   R⁴¹ is independently selected from the group: hydrogen, aryl substituted with 0-3 R⁵², C₁-C₁₀ alkyl substituted with 0-3 R⁵², and a heterocycle substituted with 0-3 R⁵²;
   R⁵² is independently selected at each occurrence from the group: a bond to Lₙ, =O, F, Cl, Br, I, -CF₃, -CN, -CO₂R⁵³, -C(=O)R⁵³, -C(=O)N(R⁵³)₂, -CHO, -CH₂OR⁵³, -OC(=O)R⁵³, -OC(=O)OR^{53a}, -OR⁵³, -OC(=O)N(R⁵³)₂, -NR⁵³C(=O)R⁵³, -NR⁵⁴C(=O)OR^{53a}, -N(R⁵³)₃⁺, -NR⁵³C(=O)N(R⁵³)₂, -NR⁵⁴SO₂N(R⁵³)₂, -NR⁵⁴SO₂R^{53a}, -SO₃H, -SO₂R^{53a}, -SR⁵³, -S(=O)R^{53a}, -SO₂N(R⁵³)₂, -N(R⁵³)₂, -NHC(=NH)NHR⁵³, -C(=NH)NHR⁵³, =NOR⁵³, NO₂, -C(=O)NHOR⁵³, -C(=O)NHNR⁵³R^{53a}, -OCH₂CO₂H, 2-(1-morpholino)ethoxy;
   R⁵³, R^{53a}, and R⁵⁴ are each independently selected at each occurrence from the group: hydrogen, C₁-C₆ alkyl, and a bond to Lₙ;
   R⁸⁰ and R⁸¹ are independently selected from the group:
      H, C₁-C₁₀ alkyl, -CN, -CO₂R⁸⁵, -C(=O)R⁸⁵, -C(=O)N(R⁸⁵)₂, C₂ - C₁₀ 1-alkene substituted with 0-3 R⁸⁴, C₂ - C₁₀ 1-alkyne substituted with 0-3 R⁸⁴, aryl substituted with 0-3 R⁸⁴, unsaturated heterocycle substituted with 0-3 R⁸⁴, and unsaturated carbocycle substituted with 0-3 R⁸⁴, provided that when one of R⁸⁰ and R⁸¹ is H or alkyl, then the other is not H or alkyl;
or, alternatively, R⁸⁰ and R⁸¹, may be taken together with the shown divalent carbon radical to form: 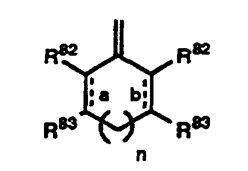 wherein:
   R⁸² and R⁸³ may be independently selected from the group:
      H, R⁸⁴, C₁-C₁₀ alkyl substituted with 0-3 R⁸⁴, C₂-C₁₀ alkenyl substituted with 0-3 R⁸⁴, C₂-C₁₀ alkynyl substituted with 0-3 R⁸⁴' aryl substituted with 0-3 R⁸⁴' heterocycle substituted with 0-3 R⁸⁴ and carbocycle substituted with 0-3 R⁸⁴;
or, alternatively, R⁸², R⁸³ may be taken together to form a fused aromatic or heterocyclic ring;
   R⁸⁴ is independently selected at each occurrence from the group: =O, F, Cl, Br, I, -CF₃, -CN, -CO₂R⁸⁵, -C(=O)R⁸⁵, -C(=O)N(R⁸⁵)₂, -N(R⁸⁵)₃+ -CH₂OR⁸⁵, -OC(=O)R⁸⁵, -OC(=O)OR^{85a}, -OR⁸⁵, -OC(=O)N(R⁸⁵)₂, -NR⁸⁵C(=O)R⁸⁵, -NR⁸⁶C(=O)OR^{85a}, -NR⁸⁵C(=O)N(R⁸⁵)₂, -NR⁸⁶SO₂N(R⁸⁵)₂, -NR⁸⁶SO₂R^{85a}, -SO₃H, -SO₂R^{85a}, -SR⁸⁵, -S(=O)R^{85a}, -SO₂N(R⁸⁵)₂, -N(R⁸⁵)₂, -NHC(=NH)NHR⁸⁵, -C(=NH)NHR⁸⁵, =NOR⁸⁵, -C(=O)NHOR⁸⁵, -OCH₂CO₂H, 2-(1-morpholino)ethoxy; and
   R⁸⁵, R^{85a}, and R⁸⁶ are each independently selected at each occurrence from the group: hydrogen and C₁-C₆ alkyl.

Preferred kits of the present invention are wherein:
A_{L1} is a tricine;
Q is a biologically active molecule selected from the group: IIb/IIIa receptor antagonists, and chemotactic peptides;
   d' is 1;
   Y¹ and Y², at each occurrence, are independently selected from:
   O, NR⁵⁶, C=O, C(=O)O, OC(=O)O, C(=O)NH, C=NR⁵⁶, NHC(=O), (NH)₂C(=O);
R⁵⁵ and R⁵⁶ are hydrogen;
R⁴⁰ is heterocycle substituted with R⁵²;
R⁴¹ is hydrogen;
R⁵² is a bond to Lₙ;
R⁸⁰ is independently selected at each occurrence from the group: -CO₂R^{85,} C₂ - C₃ 1-alkene substituted with 0-1 R⁸⁴' aryl substituted with 0-1 R⁸⁴' and unsaturated heterocycle substituted with 0-1 R⁸⁴;
R⁸¹ is H;
R⁸⁴ is independently selected at each occurrence from the group: -CO₂R⁸⁵, -OR⁸⁵,
-SO₃H, and -N(R⁸⁵)₂;
R⁸⁵ is independently selected at each occurrence from the group: hydrogen and methyl;
X¹ is independently selected at each occurrence from the group: CR⁶⁴ and N;
X² is independently selected at each occurrence from the group: CR⁶⁴, CR⁶⁴R⁶⁴, N, NR⁶⁴, and 0;
X³ is N;
Y is BR⁶⁴⁻;
provided the total number of heteroatoms in each ring of the ligand A_{L2} is 2 to 3;
R⁶⁴ is independently selected at each occurrence from the group:
   H, C₁-C₃ alkyl substituted with 0-1 R⁶⁵' aryl substituted with 0-1 R⁶⁵, and R⁶⁵;
R⁶⁵ is independently selected at each occurrence from the group: -NO₂, -CO₂R⁶⁶, -OR⁶⁶, and -SO₃H;
R⁶⁶ is hydrogen;
n is 0;
and a, b, c, d, e and f indicate the positions of optional double bonds, provided that one of e and f is a double bond.

More preferably, the present invention provides kits wherein:
AL2 is selected from the group: 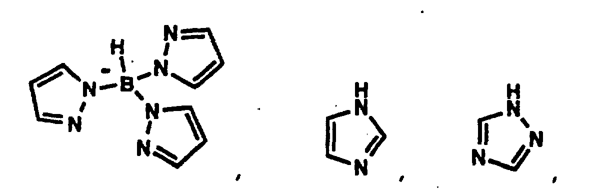 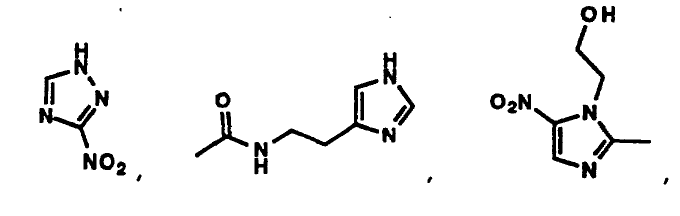 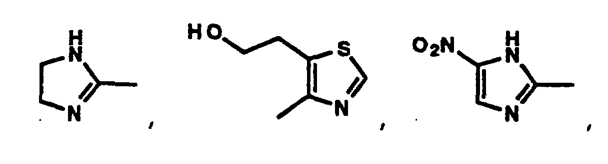 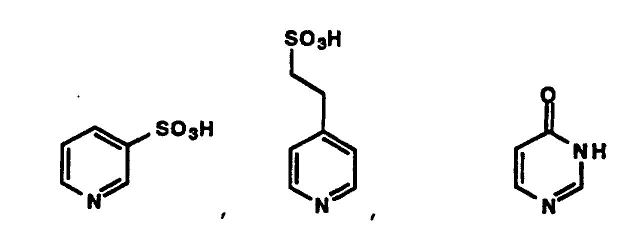 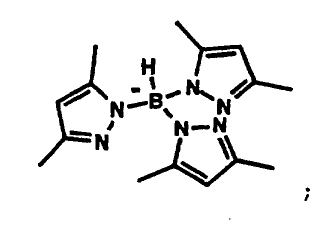
Q is 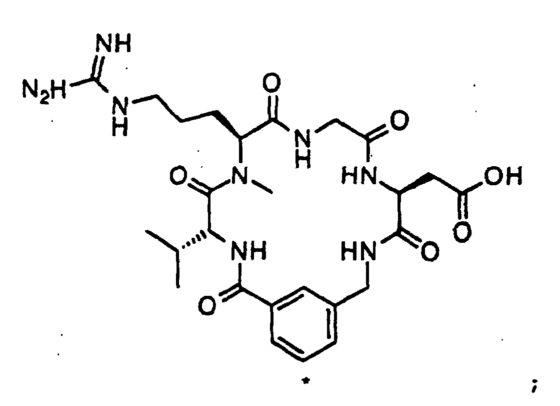
   d' is 1;
   Lₙ is attached to Q at the carbon atom designated with a * and has the formula:

      - (C=O)NH(CH₂)₅C(-O)NH-;
Cₕ is selected from R⁴⁰NNH₂- and
   R⁴⁰R⁴¹N-N=CR⁸⁰R⁸¹, wherein,
   R⁴⁰ is independently selected at each occurrence from the group: heterocycle substituted with R⁵²;
   R⁴¹ is hydrogen;
   R⁵² is a bond to Lₙ;
   R⁸⁰ is independently selected at each occurrence from the group: -CO₂R⁸⁵' C₂ - C₃ 1-alkene substituted with 0-1 R⁸⁴' aryl substituted with 0-1 R⁸⁴ and unsaturated heterocycle substituted with 0-1 R⁸⁴;
   R⁸¹ is H;
   R⁸⁴ is independently selected at each occurrence from the group: -CO₂R⁸⁵, -OR⁸⁵, -SO₃H, and -N(R⁸⁵)₂;
   R⁸⁵ is independently selected at each occurrence from the group: hydrogen and methyl.

When any variable occurs more than one time in any constituent or in any formula, its definition on each occurrence is independent of its definition at every other occurrence. Thus, for example, if a group is shown to be substituted with 0-2 R⁵², then said group may optionally be substituted with up to two R⁵² and R⁵² at each occurrence is selected independently from the defined list of possible R⁵². Also, by way of example, for the group -N(R⁵³)₂, each of the two R⁵³ substituents on N is independently selected from the defined list of possible R⁵³. Combinations of substituents and/or variables are permissible only if such combinations result in stable compounds.

By "stable compound" or "stable structure" is meant herein a compound that is sufficiently robust to survive isolation to a useful degree of purity from a reaction mixture, and formulation into an efficacious diagnostic agent.

The term "capable of stabilizing", as used herein to describe the second ancillary ligand A_{L2}, means that the ligand is capable of coordinating to the transition metal radionuclide in the presence of the first ancillary ligand and the transition metal chelator, under the conditions specified herein, resulting in a radiopharmaceutical of Formula 1 having a minimal number of isomeric forms, the relative ratios of which do not change significantly with time, and that remains substantially intact upon dilution.

The term "substituted", as used herein, means that one or more hydrogens on the designated atom or group is replaced with a selection from the indicated group, provided that the designated atom's or group's normal valency is not exceeded, and that the substitution results in a stable compound. When a substituent is keto (i.e., =O), then 2 hydrogens on the atom are replaced.

The term "bond", as used herein, means either a single or double bond.

The term "salt", as used herein, is used as defined in the CRC Handbook of Chemistry and Physics, 65th Edition, CRC Press, Boca Raton, Fla, 1984, as any substance which yields ions, other than hydrogen or hydroxyl ions.

As used herein, "alkyl" is intended to include both branched and straight-chain saturated aliphatic hydrocarbon groups having the specified number of carbon atoms; "cycloalkyl" or "carbocycle" is intended to include saturated and partially unsaturated ring groups, including mono-,bi- or poly-cyclic ring systems, such as cyclopropyl, cyclobutyl, cyclopentyl, cyclohexyl, cycloheptyl, cyclooctyl and adamantyl; "bicycloalkyl" is intended to include saturated bicyclic ring groups such as [3.3.0]bicyclooctane, [4.3.0]bicyclononane, [4.4.0]bicyclodecane (decalin), [2.2.2]bicyclooctane, and so forth.

As used herein, the term "alkene" or "alkenyl" is intended to include both branched and straight-chain groups of the formula CₙH₂ₙ₋₁ having the specified number of carbon atoms.

As used herein, the term "alkyne" or "alkynyl" is intended to include both branched and straight-chain groups of the formula CₙH₂ₙ₋₃ having the specified number of carbon atoms.

As used herein, "aryl" or "aromatic residue" is intended to mean phenyl or naphthyl, which when substituted, the substitution can be at any position.

As used herein, the term "heterocycle" or "heterocyclic ring system" is intended to mean a stable 5- to 7- membered monocyclic or bicyclic or 7- to 10-membered bicyclic heterocyclic ring which may be saturated, partially unsaturated, or aromatic, and which consists of carbon atoms and from 1 to 4 heteroatoms selected independently from the group consisting of N, O and S and wherein the nitrogen and sulfur heteroatoms may optionally be oxidized, and the nitrogen may optionally be quaternized, and including any bicyclic group in which any of the above-defined heterocyclic rings is fused to a benzene ring. The heterocyclic ring may be attached to its pendant group at any heteroatom or carbon atom which results in a stable structure. The heterocyclic rings described herein may be substituted on carbon or on a nitrogen atom if the resulting compound is stable. Examples of such heterocycles include, but are not limited to, benzopyranyl, thiadiazine, tetrazolyl, benzofuranyl, benzothiophenyl, indolene, quinoline, isoquinolinyl or benzimidazolyl, piperidinyl, 4-piperidone, 2-pyrrolidone, tetrahydrofuran, tetrahydroquinoline, tetrahydroisoquinoline, decahydroquinoline, octahydroisoquinoline, azocine, triazine (including 1,2,3-, 1,2,4-, and 1,3,5-triazine), 6*H*-1,2,5-thiadiazine, 2*H*,6*H*-1,5,2-dithiazine, thiophene, tetrahydrothiophene, thianthrene, furan, pyran, isobenzofuran, chromene, xanthene, phenoxathiin, 2*H*-pyrrole, pyrrole, imidazole, pyrazole, thiazole, isothiazole, oxazole (including 1,2,4- and 1,3,4-oxazole), isoxazole, triazole, pyridine, pyrazine, pyrimidine, pyridazine, indolizine, isoindole, 3*H*-indole, indole, 1*H*-indazole, purine, 4*H*-quinolizine, isoquinoline, quinoline, phthalazine, naphthyridine, quinoxaline, quinazoline, cinnoline, pteridine, 4a*H*-carbazole, carbazole, β-carboline, phenanthridine, acridine, perimidine, phenanthroline, phenazine, phenarsazine, phenothiazine, furazan, phenoxazine, isochroman, chroman, pyrrolidine, pyrroline, imidazolidine, imidazoline, pyrazolidine, pyrazoline, piperazine, indoline, isoindoline, quinuclidine, or morpholine. Also included are fused ring and spiro compounds containing, for example, the above heterocycles.

As used herein, the term "alkaryl" means an aryl group bearing an alkyl group of 1-10 carbon atoms; the term "aralkyl" means an alkyl group of 1-10 carbon atoms bearing an aryl group; the term "arylalkaryl" means an aryl group bearing an alkyl group of 1-10 carbon atoms bearing an aryl group; and the term "heterocycloalkyl" means an alkyl group of 1-10 carbon atoms bearing a heterocycle.

A "reducing agent" is a compound that reacts with the radionuclide, which is typically obtained as a relatively unreactive, high oxidation state compound, to lower its oxidation state by transfering electron(s) to the radionuclide, thereby making it more reactive. Reducing agents useful in the preparation of radiopharmaceuticals and in diagnostic kits useful for the preparation of said radiopharmaceuticals include but are not limited to stannous chloride, stannous fluoride, formamidine sulfinic acid, ascorbic acid, cysteine, phosphines, and cuprous or ferrous salts. Other reducing agents are described in Brodack et. al., PCT Application 94/22496, which is incorporated herein by reference.

A "transfer ligand" is a ligand that forms an intermediate complex with the radionuclide that is stable enough to prevent unwanted side-reactions but labile enough to be converted to the radiopharmaceutical. The formation of the intermediate complex is kinetically favored while the formation of the radiopharmaceutical is thermodynamically favored. Transfer ligands useful in the preparation of radiopharmaceuticals and in diagnostic kits useful for the preparation of said radiopharmaceuticals include but are not limited to gluconate, glucoheptonate, mannitol, glucarate, N,N,N',N'-ethylenediaminetetraacetic acid, pyrophosphate and methylenediphosphonate. In general, transfer ligands are comprised of oxygen or nitrogen donor atoms.

The term "donor atom" refers to the atom directly attached to a metal by a chemical bond.

"Ancillary" or "co-ligands" are ligands that are incorporated into the radiopharmaceutical during its synthesis. They serve to complete the coordination sphere of the radionuclide together with the chelator or radionuclide bonding unit of the reagent. For radiopharmaceuticals comprised of a binary ligand system, the radionuclide coordination sphere is composed of one or more chelators or bonding units from one or more reagents and one or more ancillary or co-ligands, provided that there are a total of two types of ligands, chelators or bonding units. For example, a radiopharmaceutical comprised of one chelator or bonding unit from one reagent and two of the same ancillary or co-ligands and a radiopharmaceutical comprised of two chelators or bonding units from one or two reagents and one ancillary or co-ligand are both considered to be comprised of binary ligand systems. For radiopharmaceuticals comprised of a ternary ligand system, the radionuclide coordination sphere is composed of one or more chelators or bonding units from one or more reagents and one or more of two different types of ancillary or co-ligands, provided that there are a total of three types of ligands, chelators or bonding units. For example, a radiopharmaceutical comprised of one chelator or bonding unit from one reagent and two different ancillary or co-ligands is considered to be comprised of a ternary ligand system.

Ancillary or co-ligands useful in the preparation of radiopharmaceuticals and in diagnostic kits useful for the preparation of said radiopharmaceuticals are comprised of one or more oxygen, nitrogen, carbon, sulfur, phosphorus, arsenic, selenium, and tellurium donor atoms. A ligand can be a transfer ligand in the synthesis of a radiopharmaceutical and also serve as an ancillary or co-ligand in another radiopharmaceutical. Whether a ligand is termed a transfer or ancillary or co-ligand depends on whether the ligand remains in the radionuclide coordination sphere in the radiopharmaceutical, which is determined by the coordination chemistry of the radionuclide and the chelator or bonding unit of the reagent or reagents.

A "chelator" or "bonding unit" is the moiety or group on a reagent that binds to a metal radionuclide through the formation of chemical bonds with one or more donor atoms.

The term "binding site" means the site in vivo or in vitro that binds a biologically active molecule.

A "diagnostic kit" comprises a collection of components, termed the formulation, in one or more vials which are used by the practising end user in a clinical or pharmacy setting to synthesize the radiopharmaceutical. The kit provides all the requisite components to synthesize and use the radiopharmaceutical except those that are commonly available to the practising end user, such as water or saline for injection, a solution of the radionuclide, equipment for heating the kit during the synthesis of the radiopharmaceutical if required, equipment necessary for administering the radiopharmaceutical to the patient such as syringes and shielding, and imaging equipment.

A "buffer" is a compound that is used to control the pH of the kit during its manufacture and during the synthesis of the radiopharmaceutical.

A "lyophilization aid" is a component that has favorable physical properties for lyophilization, such as the glass transition temperature, and is added to the diagnostic kit to improve the physical properties of the combination of all the components of the kit for lyophilizacion.

A "stabilization aid" is a component that is added to the radiopharmaceutical or to the diagnostic kit either to stabilize the radiopharmaceutical once it is synthesized or to prolong the shelf-life of the kit before it must be used. Stabilization aids can be antioxidants, reducing agents or radical scavengers and can provide improved stability by reacting preferentially with species that degrade other components or the radiopharmaceutical.

A "solubilization aid" is a component that improves the solubility of one or more other components in the medium required for the synthesis of the radiopharmaceutical.

A "bacteriostat" is a component that inhibits the growth of bacteria in the diagnostic kit either during its storage before use of after the kit is used to synthesize the radiopharmaceutical.

The radiopharmaceuticals of the present invention are of the formula [(Q)_{d'}Lₙ-C_{h'}]ₓ-Mₜ(A_{L1})_{y}(A_{L2})_{z}, wherein Q is a biologically active group, d' is an integer from 1 to 20, Lₙ is an optional linking group, C_{h'} is a radionuclide metal chelator or bonding unit bound to the transition metal radionuclide, Mₜ, of the formulae R⁴⁰N=N⁺=, R⁴⁰R⁴¹N-N=, or R⁴⁰N=N(H)-, A_{L1} is a first ancillary or co-ligand, A_{L2} is a second ancillary or co-ligand, x, y and z are independently 1 or 2.

The biologically active molecule Q can be a protein, antibody, antibody fragment, peptide or polypeptide, or peptidomimetic that is comprised of a recognition sequence or unit for a receptor or binding site expressed at the site of the disease, or for a receptor or binding site expressed on platelets or leukocytes. The exact chemical composition of Q is selected based on the disease state to be diagnosed, the mechanism of localization to be utilized, and to provide an optimium combination of rates of localization, clearance and radionuclidic decay.

For the purposes of this invention, the term thromboembolic disease is taken to include both venous and arterial disorders and pulmonary embolism, resulting from the formation of blood clots.

For the diagnosis of thromboembolic disorders or atherosclerosis, Q is selected from the group including the cyclic IIb/IIIa receptor antagonist compounds described in co-pending U.S. Ser. No.08/218,861 (equivalent to WO 94/22494); the RGD containing peptides described in U.S. Patents 4,578,079, 4,792,525, the applications PCT US88/04403, PCT US89/01742, PCT US90/03788, PCT US91/02356 and by Ojima et. al., 204th Meeting of the Amer. Chem. Soc., 1992, Abstract 44; the peptides that are fibrinogen receptor antagonists described in European Patent Applications 90202015.5, 90202030.4, 90202032.2, 90202032.0, 90311148.2, 90311151.6, 90311537.6, the specific binding peptides and polypeptides described as IIb/IIIa receptor ligands, ligands for the polymerization site of fibrin, laminin derivatives, ligands for fibrinogen, or thrombin ligands in PCT WO 93/23085 (excluding the technetium binding groups); the oligopeptides that correspond to the IIIa protein described in PCT WO90/00178; the hirudin-based peptides described in PCT WO90/03391; the IIb/IIIa receptor ligands described in PCT WO90/15818; the thrombus, platelet binding or atherosclerotic plaque binding peptides described in PCT WO92/13572 (excluding the technetium binding group) or GB 9313965.7; the fibrin binding peptides described in U.S. Patents 4,427,646 and 5,270,030; the hirudin-based peptides described in U.S. Patent 5,279,812; or the fibrin binding proteins described in U.S. Patent 5,217,705; the guanine derivatives that bind to the IIb/IIIa receptor described in U.S. Patent 5,086,069; or the tyrosine derivatives described in European Patent Application 0478328A1, and by Hartman et. al., J. Med. Chem., 1992, 35, 4640; or oxidized low density lipoprotein (LDL).

For the diagnosis of infection, inflammation or transplant rejection, Q is selected from the group including the leukocyte binding peptides described in PCT WO93/17719 (excluding the technetium binding group), PCT WO92/13572 (excluding the technetium binding group) or U.S. Ser. No. 08/140000; the chemotactic peptides described in Eur. Pat. Appl. 90108734.6 or A. Fischman et. al., Semin. Nuc. Med., 1994, 24, 154; or the leukostimulatory agents described in U.S. Patent 5,277,892.

For the diagnosis of cancer, Q is selected from the group of somatostatin analogs described in UK Application 8927255.3 or PCT WO94/00489, the selectin binding peptides described in PCT WO94/05269, the biological-function domains described in PCT WO93/12819, Platelet Factor 4 or the growth factors (PDGF, EGF, FGF, TNF, MCSF or Il-8).

Q may also represent proteins, antibodies, antibody fragments, peptides, polypeptides, or peptidomimetics that bind to receptors or binding sites on other tissues, organs, enzymes or fluids. Examples include the β-amyloid proteins that have been demonstrated to accumulate in patients with Alzheimer's disease, atrial naturetic factor derived peptides that bind to myocardial and renal receptors, antimyosin antibodies that bind to areas of infarcted tissues, or nitroimidazole derivatives that localize in hypoxic areas in vivo.

The group C_{h'} is termed a hydrazido (of formula R⁴⁰R⁴¹N-N=), or diazenido (of formula R⁴⁰N=N⁺= or R⁴⁰N=N(H)-) group and serves as the point of attachment of the radionuclide to the remainder of the radiopharmaceutical designated by the formula (Q)_{d'}-Lₙ or (Q)_{d'}. A diazenido group can be either terminal (only one atom of the group is bound to the radionuclide) or chelating. In order to have a chelating diazenido group at least one other atom of the group, located on R⁴⁰, must also be bound to the radionuclide. The atoms bound to the metal are termed donor atoms.

The transition metal radionuclide, Mₜ, is selected from the group: technetium-99m, rhenium-186 and rhenium-188. For diagnostic purposes Tc-99m is the preferred isotope. Its 6 hour half-life and 140 keV gamma ray emission energy are almost ideal for gamma scintigraphy using equipment and procedures well established for those skilled in the art. The rhenium isotopes also have gamma ray emission energies that are compatible with gamma scintigraphy, however, they also emit high energy beta particles that are more damaging to living tissues. These beta particle emissions can be utilized for therapeutic purposes, for example, cancer radiotherapy.

The coordination sphere of the radionuclide includes all the ligands or groups bound to the radionuclide. For a transition metal radionuclide, Mₜ, to be stable it typically has a coordination number (number of donor atoms) comprised of an integer greater than or equal to 4 and less than or equal to 8; that is there are 4 to 8 atoms bound to the metal and it is said to have a complete coordination sphere. The requisite coordination number for a stable radionuclide complex is determined by the identity of the radionuclide, its oxidation state, and the type of donor atoms. If the chelator or bonding unit C_{h'} does not provide all of the atoms necessary to stabilize the metal radionuclide by completing its coordination sphere, the coordination sphere is completed by donor atoms from other ligands, termed ancillary or co-ligands, which can also be either terminal or chelating.

A large number of ligands can serve as ancillary or co-ligands, the choice of which is determined by a variety of considerations such as the ease of synthesis of the radiopharmaceutical, the chemical and physical properties of the ancillary ligand, the rate of formation, the yield, and the number of isomeric forms of the resulting radiopharmaceuticals, the ability to administer said ancillary or co-ligand to a patient without adverse physiological consequences to said patient, and the compatibility of the ligand in a lyophilized kit formulation. The charge and lipophilicity of the ancillary ligand will effect the charge and lipophilicity of the radiopharmaceuticals. For example, the use of 4,5-dihydroxy-1,3-benzene disulfonate results in radiopharmaceuticals with an additional two anionic groups because the sulfonate groups will be anionic under physiological conditions. The use of N-alkyl substituted 3,4-hydroxypyridinones results in radiopharmaceuticals with varying degrees of lipophilicity depending on the size of the alkyl substituents.

The radiopharmaceuticals of the present invention are comprised of two types of ancillary or co-ligands designated A_{L1} and A_{L2}. Ancillary ligands A_{L1} are comprised of two or more hard donor atoms such as oxygen and amine nitrogen (sp³ hydribidized). The donor atoms occupy at least two of the sites in the coordination sphere of the radionuclide metal, Mₜ; the ancillary ligand A_{L1} serves as one of the three ligands in the ternary ligand system. Examples of ancillary ligands A_{L1} include but are not limited to dioxygen ligands and functionalized aminocarboxylates. A large number of such ligands are available from commercial sources.

Ancillary dioxygen ligands include ligands that coordinate to the metal ion through at least two oxygen donor atoms. Examples include but are not limited to: glucoheptonate, gluconate, 2-hydroxyisobutyrate, lactate, tartrate, mannitol, glucarate, maltol, Kojic acid, 2,2-bis(hydroxymethyl)propionic acid, 4,5-dihydroxy-1,3-benzene disulfonate, or substituted or unsubstituted 1,2 or 3,4 hydroxypyridinones. (The names for the ligands in these examples refer to either the protonated or non-protonated forms of the ligands.)

Functionalized aminocarboxylates include ligands that have a combination of amine nitrogen and oxygen donor atoms. Examples include but are not limited to: iminodiacetic acid, 2,3-diaminopropionic acid, nitrilotriacetic acid, N,N'-ethylenediamine diacetic acid, N,N,N'-ethylenediamine triacetic acid, hydroxyethylethylenediamine triacetic acid, and N,N'-ethylenediamine bis-hydroxyphenylglycine. (The names for the ligands in these examples refer to either the protonated or non-protonated forms of the ligands.)

A series of functionalized aminocarboxylates are disclosed by Bridger et. al. in U.S. Patent 5,350,837, herein incorporated by reference, that result in improved rates of formation of technetium labeled hydrazino modified proteins. We have determined that certain of these aminocarboxylates result in improved yields of the radiopharmaceuticals of the present invention. The preferred ancillary ligands A_{L1} functionalized aminocarboxylates that are derivatives of glycine; the most preferred is tricine (tris(hydroxymethyl)methylglycine).

The second type of ancillary ligands A_{L2} are comprised of one or more soft donor atoms selected from the group: imine nitrogen (sp² hydridized), sulfur (sp² hydridized) and carbon (sp hybridized); atoms which have π-acid character. Ligands A_{L2} can be monodentate, bidentate or tridentate, the denticity is defined by the number of donor atoms in the ligand. One of the two donor atoms in a bidentate ligand and one of the three donor atoms in a tridentate ligand must be a soft donor atom.

The ligands A_{L2} that are comprised of imine nitrogen are unsaturated or aromatic nitrogen-containing, 5 or 6-membered heterocycles. The ligands that are comprised of sulfur (sp² hybridized) donor atoms are thiocarbonyls, comprised of the moiety C=S. The ligands comprised of carbon (sp hybridized) donor atoms are isonitriles, comprised of the moiety CNR, where R is an organic radical. A large number of such ligands are available from commercial sources. Isonitriles can be synthesized as described in European Patent 0107734 and in U. S. Patent 5,279,811, herein incorporated by reference. Preferred ancillary ligands A_{L2} are unsaturated or aromatic 5 or 6 membered heterocycles. The most preferred ancillary ligands A_{L2} are unsaturated 5 membered heterocycles.

The ancillary ligands A_{L2} may be substituted with alkyl, aryl, alkoxy, heterocycle, aralkyl, alkaryl and arylalkaryl groups and may or may not bear functional groups comprised of heteroatoms such as oxygen, nitrogen, phosphorus or sulfur. Examples of such functional groups include but are not limited to: hydroxyl, carboxyl, carboxamide, nitro, ether, ketone, amino, ammonium, sulfonate, sulfonamide, phosphonate, and phosphonamide. The functional groups may be chosen to alter the lipophilicity and water solubility of the ligands which may affect the biological properties of the radiopharmaceuticals, such as altering the distribution into non-target tissues, cells or fluids, and the mechanism and rate of elimination from the body.

The radiopharmaceuticals of the present invention can be easily prepared by admixing a salt of a radionuclide, a reagent of Formula 2, an ancillary ligand A_{L1}, an ancillary ligand A_{L2}, and a reducing agent, in an aqueous solution at temperatures from room temperature to 100 °C.

**(Q) d'L**_{**n**}**-C**_{**h**} (2)

and pharmaceutically acceptable salts thereof, wherein: Q, d', Lₙ are as defined above, Cₕ is a radionuclide metal chelator selected from the group: R⁴⁰R⁴¹N-N=C(C₁-C₃ alkyl)₂ and R⁴⁰NNH₂-, and R⁴⁰R⁴¹N-N=C(R⁸⁰)(R⁸¹), and pharmaceutically acceptable salts thereof. The synthesis of reagents of formula 2 is described in co-pending U.S.S.N. 08/218,861 (equivalent to WO 94/22494) and in co-pending U.S.S.N. 08/476,296.

When Cₕ is a hydrazone group, then it must first be converted to a hydrazine of formula R⁴⁰R⁴¹NNH², which may or may not be protonated, prior to complexation with the metal radionuclide, Mₜ. The chelator or bonding unit, Cₕ, when bound to the metal radionuclide, Mₜ, is designated Cₕ'. The conversion of the hydrazone group to the hydrazine can occur either prior to reaction with the radionuclide, in which case the radionuclide and the ancillary or co-ligand or ligands are combined not with the reagent but with a hydrolyzed form of the reagent bearing the chelator or bonding unit, Cₕ, or in the presence of the radionuclide in which case the reagent itself is combined with the radionuclide and the ancillary or co-ligand or ligands. In the latter case, the pH of the reaction mixture must be neutral or acidic.

Alternatively, the radiopharmaceuticals of the present invention can be prepared by first admixing a salt of a radionuclide, an ancillary ligand A_{L1}, and a reducing agent in an aqueous solution at temperatures from room temperature to 100 °C to form an intermediate radionuclide complex with the ancillary ligand A_{L1} then adding a reagent of Formula 2 and an ancillary ligand A_{L2} and reacting further at temperatures from room temperature to 100 °C.

Alternatively, the radiopharmaceuticals of the present invention can be prepared by first admixing a salt of a radionuclide, an ancillary ligand A_{L1}, a reagent of Formula 2, and a reducing agent in an aqueous solution at temperatures from room temperature to 100 °C to form an intermediate radionuclide complex, and then adding an ancillary ligand A_{L2} and reacting further at temperatures from room temperature to 100 °C.

The total time of preparation will vary depending on the identity of the radionuclide, the identities and amounts of the reactants and the procedure used for the preparation. The preparations may be complete, resulting in > 80% yield of the radiopharmaceutical, in 1 minute or may require more time. If higher purity radiopharmaceuticals are needed or desired, the products can be purified by any of a number of techniques well known to those skilled in the art such as liquid chromatography, solid phase extraction, solvent extraction, dialysis or ultrafiltration.

The technetium and rhenium radionuclides are preferably in the chemical form of pertechnetate or perrhenate and a pharmaceutically acceptable cation. The pertechnetate salt form is preferably sodium pertechnetate such as obtained from commercial Tc-99m generators. The amount of pertechnetate used to prepare the radiopharmaceuticals of the present invention can range from 0.1 mCi to 1 Ci, or more preferably from 1 to 200 mCi.

The amount of the reagent of formula 2 used to prepare the radiopharmaceuticals of the present invention can range from 0.1 µg to 10 mg, or more preferably from 0.5 µg to 100 µg. The amount used will be dictated by the amounts of the other reactants and the identity of the radiopharmaceuticals of Formula 1 to be prepared.

The amounts of the ancillary ligands A_{L1} used can range from 0.1 mg to 1 g, or more preferrably from 1 mg to 100 mg. The exact amount for a particular radiopharmaceutical is a function of identity of the radiopharmaceuticals of Formula 1 to be prepared, the procedure used and the amounts and identities of the other reactants. Too large an amount of A_{L1} will result in the formation of by-products comprised of technetium labeled A_{L1} without a biologically active molecule or by-products comprised of technetium labeled biologically active molecules with the ancillary ligand A_{L1} but without the ancillary ligand A_{L2}. Too small an amount of A_{L1} will result in other by-products such as technetium labeled biologically active molecules with the ancillary ligand A_{L2} but without the ancillary ligand A_{L1}, or reduced hydrolyzed technetium, or technetium colloid.

The amounts of the ancillary ligands A_{L2} used can range from 0.001 mg to 1 g, or more preferrably from 0.01 mg to 10 mg. The exact amount for a particular radiopharmaceutical is a function of the identity of the radiopharmaceuticals of Formula 1 to be prepared, the procedure used and the amounts and identities of the other reactants. Too large an amount of A_{L2} will result in the formation of by-products comprised of technetium labeled A_{L2} without a biologically active molecule or by-products comprised of technetium labeled biologically active molecules with the ancillary ligand A_{L2} but without the ancillary ligand A_{L1}. If the moiety (Q)_{d'}-Lₙ-C_{h'} bears one or more substituents that are comprised of a soft donor atom, as defined above, at least a tenfold molar excess of the ancillary ligand A_{L2} to the reagent of formula 2 is required to prevent the substituent from interfering with the coordination of the ancillary ligand A_{L2} to the metal radionuclide, Mₜ.

Suitable reducing agents for the synthesis of the radiopharmaceuticals of the present invention include stannous salts, dithionite or bisulfite salts, borohydride salts, and formamidinesulfinic acid, wherein the salts are of any pharmaceutically acceptable form. The preferred reducing agent is a stannous salt. The amount of a reducing agent used can range from 0.001 mg to 10 mg, or more preferably from 0.005 mg to 1 mg.

The specific structure of a radiopharmaceutical of the present invention will depend on the identity of the biologically active molecule Q, the number d', the identity of the linker Lₙ, the identity of the chelator moiety C_{h'}, the identity of the ancillary ligand A_{L1}, the identity of the ancillary ligand A_{L2}, and the identity of the radionuclide Mₜ. The identities of Q, Lₙ, and C_{h'} and the number d' are determined by the choice of the reagent of Formulae 2 or 3. For a given reagent of Formulae 2 or 3, the amount of the reagent, the amount and identity of the ancillary ligands A_{L1} and A_{L2}, the identity of the radionuclide Mₜ and the synthesis conditions employed will determine the structure of the radiopharmaceutical of Formula 1.

Radiopharmaceuticals synthesized using concentrations of reagents of Formulae 2 or 3 of <100 µg/mL, will be comprised of one hydrazido or diazenido group C_{h'}; the value of x will be 1. Those synthesized using >1 mg/mL concentrations will be comprised of two hydrazido or diazenido groups; the value of x will be 2. The two C_{h'} groups may be the same or different. For most applications, only a limited amount of the biologically active molecule can be injected and not result in undesired side-effects, such as chemical toxicity, interference with a biological process or an altered biodistibution of the radiopharmaceutical. Therefore, the radiopharmaceuticals with x equal to 2, which require higher concentrations of the reagents of Formula 2 comprised in part of the biologically active molecule, will have to be diluted or purified after synthesis to avoid such side-effects.

The identities and amounts used of the ancillary ligands A_{L1} and A_{L2} will determine the values of the variables y and z. The values of y and z can independently be an integer from 1 to 2. In combination, the values of y and z will result in a technetium coordination sphere that is made up of at least five and no more than seven donor atoms. For monodentate ancillary ligands A_{L2}, z can be an integer from 1 to 2; for bidentate or tridentate ancillary ligands A_{L2}, z is 1. The preferred combination for monodentate ligands is y equal to 1 or 2 and z equal to 1. The preferred combination for bidentate or tridentate ligands is y equal to 1 and z equal to 1.

Another aspect of the present invention are diagnostic kits for the preparation of radiopharmaceuticals useful as imaging agents for the diagnosis of cardiovascular disorders, infectious disease, inflammatory disease and cancer. Diagnostic kits of the present invention comprise one or more vials containing the sterile, non-pyrogenic, formulation comprised of a predetermined amount of the reagent of formulae (Q)_{d'}-Lₙ-Cₕ or (Q)_{d'}-Lₙ-H_{z}, one or two ancillary or co-ligands and optionally other components such as reducing agents, transfer ligands, buffers, lyophilization aids, stabilization aids, solubilization aids and bacteriostats. The inclusion of one or more optional components in the formulation will frequently improve the ease of synthesis of the radiopharmaceutical by the practising end user, the ease of manufacturing the kit, the shelf-life of the kit, or the stability and shelf-life of the radiopharmaceutical. The improvement achieved by the inclusion of an optional component in the formulation must be weighed against the added complexity of the formulation and added cost to manufacture the kit. The one or more vials that contain all or part of the formulation can independently be in the form of a sterile solution or a lyophilized solid.

Buffers useful in the preparation of radiopharmaceuticals and in diagnostic kits useful for the preparation of said radiopharmaceuticals include but are not limited to phosphate, citrate, sulfosalicylate, and acetate. A more complete list can be found in the United States Pharmacopeia.

Lyophilization aids useful in the preparation of diagnostic kits useful for the preparation of radiopharmaceuticals include but are not limited to mannitol, lactose, sorbitol, dextran, Ficoll, and polyvinylpyrrolidine(PVP).

Stabilization aids useful in the preparation of radiopharmaceuticals and in diagnostic kits useful for the preparation of said radiopharmaceuticals include but are not limited to ascorbic acid, cysteine, monothioglycerol, sodium bisulfite, sodium metabisulfite, gentisic acid, and inositol.

Solubilization aids useful in the preparation of radiopharmaceuticals and in diagnostic kits useful for the preparation of said radiopharmaceuticals include but are not limited to ethanol, glycerin, polyethylene glycol, propylene glycol, polyoxyethylene sorbitan monooleate, sorbitan monoloeate, polysorbates, poly(oxyethylene)poly(oxypropylene)poly(oxyethylene) block copolymers (Pluronics) and lecithin. Preferred solubilizing aids are polyethylene glycol, and Pluronics.

Bacteriostats useful in the preparation of radiopharmaceuticals and in diagnostic kits useful for the preparation of said radiopharmaceuticals include but are not limited to benzyl alcohol, benzalkonium chloride, chlorbutanol, and methyl, propyl or butyl paraben.

A component in a diagnostic kit can also serve more than one function. A reducing agent can also serve as a stabilization aid, a buffer can also serve as a transfer ligand, a lyophilization aid can also serve as a transfer, ancillary or co-ligand and so forth.

The predetermined amounts of each component in the formulation are determined by a variety of considerations that are in some cases specific for that component and in other cases dependent on the amount of another component or the presence and amount of an optional component. In general, the minimal amount of each component is used that will give the desired effect of the formulation. The desired effect of the formulation is that the practising end user can synthesize the radiopharmaceutical and have a high degree of certainty that the radiopharmaceutical can be safely injected into a patient and will provide diagnostic information about the disease state of that patient.

The diagnostic kits of the present invention will also contain written instructions for the practising end user to follow to synthesize the radiopharmaceuticals. These instructions may be affixed to one or more of the vials or to the container in which the vial or vials are packaged for shipping or may be a separate insert, termed the package insert.

The present invention may be used in a method of imaging the site of thrombotic disease in a patient involving: (1) synthesizing a radiopharmaceutical using a reagent of the present invention capable of localizing at sites of thrombotic disease due to an interaction between the biologically active group, Q, of the radiopharmaceutical and a receptor or binding site expressed at the site of the disease or with a receptor or binding site on an endogenous blood component that accumulates at the site; (2) administering said radiopharmaceutical to a patient by injection or infusion; (3) imaging the patient using either planar or SPECT gamma scintigraphy.

The present invention may also be used in a method of imaging the site of infection or infectious disease in a patient involving: (1) synthesizing a radiopharmaceutical using a reagent of the present invention capable of localizing at sites of infection or infectious disease due to an interaction between the biologically active group, Q, of the radiopharmaceutical and a receptor or binding site expressed at the site of the disease or with a receptor or binding site on an endogenous blood component that accumulates at the site; (2) administering said radiopharmaceutical to a patient by injection or infusion; (3) imaging the patient using either planar or SPECT gamma scintigraphy.

The present invention may also be used in a method of imaging the site of inflammation in a patient involving: (1) synthesizing a radiopharmaceutical using a reagent of the present invention capable of localizing at sites of inflammation due to an interaction between the biologically active group, Q, of the radiopharmaceutical and a receptor or binding site expressed at the site of inflammation or with a receptor or binding site on an endogenous blood component that accumulates at the site; (2) administering said radiopharmaceucical to a patient by injection or infusion; (3) imaging the patient using either planar or SPECT gamma scintigraphy.

The present invention may also be used in a method of imaging the site of cancer in a patient involving: (1) synthesizing a radiopharmaceutical using a reagent of the present invention capable of localizing at sites of cancer due to an interaction between the biologically active group, Q, of the radiopharmaceutical and a receptor or binding site expressed at the site of the cancer or with a receptor or binding site on an endogenous blood component that accumulates at the site; (2) administering said radiopharmaceutical to a patient by injection or infusion; (3) imaging the patient using either planar or SPECT gamma scintigraphy.

The radiopharmaceuticals are administered by intravenous injection, usually in saline solution, at a dose of 1 to 100 mCi per 70 kg body weight, or preferably at a dose of 5 to 50 mCi. Imaging is performed using known procedures.

### EXAMPLE SECTION

The materials used to synthesize the radiopharmaceuticals of the present invention described in the following examples were obtained as follows. The reagent of Formula 2, Cyclo(D-Val-NMeArg-Gly-Asp-Mamb(hydrazino-nicotinyl-5-Aca)) was synthesized as described in co-pending U.S. Ser. No. 08/218,861 (equivalent to WO 94/22494). The ancillary ligands tricine, tris(1-pyrazoyl)borohydride, imidazole, 2-methyl-5-nitroimidazole, ornidazole, metronidazole, 1,2,4-triazole, 3-nitro-1,2,4-triazole, acetyl histamine, urocanic acid, 2-methylimidazoline, 4-methyl-5-thiazoleethanol, tris(3,5-dimethyl-1-pyrazolyl)borohydride, adenosine, 8-hydroxyquinoline-5-sulfonic acid, and stannous chloride were obtained from commercial sources and used as received. Deionized water was obtained from a Milli-Q Water System and was of > 18 MΩ quality. Technetium-99m-pertechnetate (^{99m}TcO₄-) was obtained from a DuPont Pharma ⁹⁹Mo/^{99m}Tc generator.

### Example 1.

### Synthesis of ^{99m}Tc(tricine) (tris(1-pyrazolyl)borohydride)-Cyclo(D-Val-NMeArg-Gly-Asp-Mamb(hydrazino-nicotinyl-5-Aca))

To a 10 mL vial was added 0.4 mL of ^{99m}TcO₄- (100 mCi/mL) eluent, followed by 0.1 mL of Cyclo(D-Val-NMeArg-Gly-Asp-Mamb(hydrazino-nicotinyl-5-Aca)) (100 µg/mL) in saline, 0.3 mL of tricine (100 mg/mL, pH 7), and 10 µL of SnCl₂ (10 mg/mL) in 1N HCl. The reaction mixture was heated at 50 °C for 15 min. To the reaction solution above was added 0.25 mL of tris(1-pyrazolyl)borohydride (20 mg/mL) in saline. The mixture was heated at 50 °C for 30 min, and was then analyzed by HPLC Method 1.

### Example 2

### Synthesis of ^{99m}Tc(tricine)(imidazole)-Cyclo(D-Val-NMeArg-Gly-Asp-Mamb(hydrazino-nicotinyl-5-Aca))

To a 10 mL vial was added 0.3 mL of ^{99m}TcO₄-solution (100 mCi/mL) in saline, 0.4 mL of tricine solution (100 mg/mL, pH ∼ 5.0) in H₂O, 0.1 mL of Cyclo(D-Val-NMeArg-Gly-Asp-Mamb(hydrazino-nicotinyl-5-Aca))solution (100 µg/mL) in H₂O, and 10 µL of SnCl₂·2H₂O solution (10 mg/mL) in 1.0 N HCl. The reaction mixture was allowed to stand at room temperature for 15 min. After addition of 0.4 mL of imidazole solution (10 mg/mL) in H₂O, the reaction mixture was heated at 70 °C for 30 min, and was then analyzed by HPLC Method 1.

### Example 3.

### Synthesis of ^{99m}Tc(tricine)(1,2,4-triazole)-Cyclo(D-Val-NMeArg-Gly-Asp-Mamb(hydrazino-nicotinyl-5-Aca))

To a 10 mL vial was added 0.3 mL of ^{99m}TcO₄⁻ solution (100 mCi/mL) in saline, 0.4 mL of tricine solution (100 mg/mL, pH ∼ 5.0) in H₂O, 0.2 mL of Cyclo(D-Val-NMeArg-Gly-Asp-Mamb(hydrazino-nicotinyl-5-Aca)) solution (50 µg/mL) in H₂O, 0.2 mL of 3-nitro-1,2,4-triazole solution (30 mg/mL) in H₂O, and 5 µL of SnCl₂·2H₂O solution (10 mg/mL) in 1.0 N HCl. The reaction mixture was heated at 75 °C for 25 min, and was then analyzed by HPLC Method 1.

### Examnie 4.

### Synthesis of ^{99m}Tc(tricine)(3-nitro-1,2,4-triazole)-Cyclo(D-Val-NMeArg-Gly-Asp-Mamb(hydrazino-nicotinyl-5-Aca))

To a 10 mL vial was added 0.3 mL of ^{99m}TcO₄⁻ solution (100 mCi/mL) in saline, 0.4 mL of tricine solution (100 mg/mL, pH ∼ 5.0) in H₂O, 0.2 mL of Cyclo(D-Val-NMeArg-Gly-Asp-Mamb(hydrazino-nicotinyl-5-Aca)) solution (50 µg/mL) in H₂O, 0.2 mL of 3-nitro-1,2,4-triazole solution (30 mg/mL) in H₂O, and 5 µL of SnCl₂·2H₂O solution (10 mg/mL) in 1.0 N HCl. The reaction mixture was heated at 75 °C for 25 min, and was then analyzed by HPLC Method 1.

### Example 5.

### Synthesis of ^{99m}Tc(tricine)(acetyl histamine)-Cycio(D-Val-NMeArg-Gly-Asp-Mamb(hydrazino-nicotinyl-5-Aca))

To a 10 mL vial was added 0.4 mL of ^{99m}TcO₄⁻ (100 mCi/mL) eluent, followed by 0.2 mL of Cyclo(D-Val-NMeArg-Gly-Asp-Mamb(hydrazino-nicotinyl-5-Aca)) (50 µg/mL) in saline, 0.3 mL of tricine (100 mg/mL, pH 7), and 10 µL of SnCl₂ (10 mg/mL) in 1 N HCl. The reaction mixture was heated at 55 °C for 15 min. To the reaction solution above was added 0.5 mL of acetyl histamine (20 mg/mL) in H₂O. The mixture was heated at 55 °C for 60 min, and was then analyzed by HPLC Method 1.

### Example 6.

### Synthesis of ^{99m}Tc(tricine)(metronidazole)Cyclo(D-Val-NMeArg-Gly-Asp-Mamb (hydrazino-nicotinyl-5-Aca))

To a 10 mL vial was added 0.4 mL of ^{99m}TcO₄⁻ solution (100 mCi/mL) in saline, 0.3 mL of tricine solution (100 mg/mL, pH ∼ 5.0) in H₂O, 0.2 mL of Cyclo(D-Val-NMeArg-Gly-Asp-Mamb(hydrazino-nicotinyl-5-Aca)) solution (50 µg/mL) in H₂O, and 10 µL of SnCl₂·2H₂O solution (10 mg/mL) in 1.0 N HCl. The reaction mixture was heated at 50 °C for 30 min. After addition of 0.5 mL of metrornidazole solution (20 mg/mL) in H₂O, the reaction mixture was heated at 70 °C for 30 min, and was then analyzed by HPLC Method 1.

### Example 7.

### Synthesis of ^{99m}Tc(tricine)(2-methylimidazoline)-Cyclo(D-Val-NMeArg-Gly-Asp-Mamb(hydrazino-nicotinyl-5-Aca))

To a 10 mL vial was added 0.4 mL of ^{99m}TcO₄⁻ (100 mCi/mL) eluent, followed by 0.2 mL of Cyclo(D-Val-NMeArg-Gly-Asp-Mamb(hydrazino-nicotinyl-5-Aca)) (50 µg/mL) in saline, 0.3 mL of tricine (100 mg/mL, pH 7), and 10 µL of SnCl₂ (10 mg/mL) in 1N HCl. The reaction mixture was heated at 50 °C for 15 min. To the reaction solution above was added 0.5 mL of 2-methylimidazoline (20 mg/mL) in in saline. The mixture was heated at 55 °C for 60 min, and was then analyzed by HPLC Method 1.

### Example 8.

### Synthesis of ^{99m}Tc(tricine)(3-pyridinesulfonic acid)(Cyclo(D-Val-NMeArg-Gly-Asp-Mamb(hydrazinonicotinyl-5-Aca))

To a 10 mL vial was added 0.4 mL of tricine solution (100 mg/mL, pH 5.0) in H₂O, 0.4 mL of Cyclo(D-Val-NMeArg-Gly-Asp-Mamb(hydrazino-nicotinyl-5-Aca) solution (50 µg/mL) in H₂O, 0.2 mL of 3-pyridinesulfonic acid solution (10 mg/mL) in H₂O, 0.3 mL of ^{99m}TcO₄-solution (100 mCi/mL) in saline, and 25 µL of SnCl₂·2H₂O solution (10 mg/mL) in 0.1 N HCl.
The mixture was heated at 80 °C for 20 min, and was then analyzed by HPLC Method 2.

### Example 9.

### Synthesis of ^{99m}Tc(tricine)(4-methyl-5-thiazoleethanol)-Cyclo(D-Val-NMeArg-Gly-Asp-Mamb(hydrazino-nicotinyl-5-Aca))

To a 10 mL vial was added 0.4 mL of ^{99m}TcO₄⁻ (100 mCi/mL) eluent, followed by 0.2 mL of Cyclo(D-Val-NMeArg-Gly-Asp-Mamb(hydrazino-nicotinyl-5-Aca)) (50 µg/mL) in saline, 0.3 mL of tricine (100 mg/mL, pH 7), and 10 µL of SnCl₂ (10 mg/mL) in 1N HCl. The reaction mixture was heated at 50 °C for 30 min. To the reaction solution above was added 0.5 mL of 4-methylthiazoleethanol (10 mg/mL) in in saline. The mixture was heated at 75 °C for 30 min, and was then analyzed byHPLC Method 1.

### Example 10.

### Synthesis of ^{99m}Tc(tricine)(tris(3,5-dimethylpyrazolyl) borohydride)-Cyclo(D-Val-NMeArg-Gly-Asp-Mamb(hydrazino-nicotinyl-5-Aca))

To a 10 mL vial was added 0.2 mL of ^{99m}TcO₄- (100 mCi/mL) eluent, followed by 0.2 mL of Cyclo(D-Val-NMeArg-Gly-Asp-Mamb(hydrazino-nicotinyl-5-Aca)) (50 µg/mL) in saline, 0.4 mL of tricine (100 mg/mL, pH 7), 0.2 mL of tris(3,5-dimethylpyrazoly)borohydride (20 mg/mL) in saline, and 10 µL of SnCl₂ (10 mg/mL) in 1N HCl. The mixture was heated at 75 °C for 30 min, and was then analyzed by HPLC Method 1.

### Example 11.

### Synthesis of ^{99m}Tc(tricine)(4-pyridineethanesulfonic acid)(Cyclo(D-Val-NMeArg-Gly-Asp-Mamb(hydrazinonicotinyl-5-Aca))

To a 10 mL vial was added 0.4 mL of tricine solution (100 mg/mL, pH 5.0) in H₂O, 0.4 mL of Cyclo(D-Val-NMeArg-Gly-Asp-Mamb(hydrazino-nicotinyl-5-Aca)) (50 µg/mL) in H₂O, 0.4 mL of 4-pyridineethanesulfonic acid solution (35 mg/mL) in H₂O, 0.2 mL of ^{99m}TcO₄⁻ solution (250 mCi/mL) in saline, and 25 µL of SnCl₂·2H₂O solution (10 mg/mL) in 0.1 N HCl. The reaction mixture was heated at 80 °C for 20 min, and was then analyzed by HPLC Method 1.

### Example 12.

### Synthesis of ^{99m}Tc(tricine)(ornidazole)-Cyclo(D-Val-NMeArg-Gly-Asp-Mamb(hydrazino-nicotinyl-5-Aca))

To a 10 mL vial was added 0.4 mL of ^{99m}TcO₄⁻ solution (100 mCi/mL) in saline, 0.2 mL of tricine solution (100 mg/mL, pH ∼ 5.0) in H₂O, 0.2 mL of Cyclo(D-Val-NMeArg-Gly-Asp-Mamb(hydrazino-nicotinyl-5-Aca)) solution (50 µg/mL) in H₂O, and 10 µL of SnCl₂·2H₂O solution (10 mg/mL) in 1.0 N HCl. The reaction mixture was allowed to stand at room temperature for 20 min. After addition of 0.5 mL of ornidazole solution (20 mg/mL) in H₂O, the reaction mixture was heated at 70 °C for 30 min, and was then analyzed by HPLC Method 1.

### Example 13.

### Synthesis of ^{99m}Tc(tricine)(4-(3H)pyrimidone)(Cyclo(D-Val-NMeArg-Gly-Asp-Mamb(hydrazino-nicotinyl-5-Aca))

To a 10 mL vial was added 0.4 mL of tricine solution (100 mg/mL, pH 5.0) in H₂O, 0.4 mL of Cyclo(D-Val-NMeArg-Gly-Asp-Mamb(hydrazino-nicotinyl-5-Aca) (50 µg/mL) in H₂O, 0.2 mL of 4-(3H)pyrimidone solution (10 mg/mL) in H₂O, 0.3 mL of ^{99m}TcO₄⁻ solution (100 mCi/mL) in saline, and 25 µL of SnCl₂·2H₂O solution (10 mg/mL) in 0.1 N HCl. The reaction mixture was heated at 80 °C for 20 min, and was then analyzed by HPLC Method 2.

### Example 14.

### Synthesis of ^{99m}Tc(tricine)(2-methyl-5-nitroimidazole)-Cyclo(D-Val-NMeArg-Gly-Asp-Mamb(hydrazino-nicotinyl-5-Aca))

To a 10 mL vial was added 0.4 mL of ^{99m}TcO₄- solution (100 mCi/mL) in saline, 0.2 mL of tricine solution (100 mg/mL, pH ∼ 5.0) in H₂O, 0.2 mL of Cyclo(D-Val-NMeArg-Gly-Asp-Mamb(hydrazino-nicotinyl-5-Aca)) solution (50 µg/mL) in H₂O, and 10 µL of SnCl₂·2H₂O solution (10 mg/mL) in 1.0 N HCl. The reaction mixture was allowed to stand at room temperature for 15 min. After addition of 0.2 mL of 2-methyl-5-nitroimidazole solution (10 mg/mL) in H₂O, the reaction mixture was heated at 70 °C for 30 min, and was then analyzed by HPLC Method 1.

**Table 1**

| Analytical and Yield Data on Ternary Complexes | | |
|---|---|---|
| Example | Retention Time | % Yield |
| | (min) | |
| 1 | 11.8 | 91 |
| 2 | 12.1, 12.5 | 86 |
| 3 | 12.7, 12.9 | 99 |
| 4 | 13.0, 13.2 | 95 |
| 5 | 12.6 | 97 |
| 6 | 14.4 | 89 |
| 7 | 12.3 | 97 |
| 8 | 8.8* | 91 |
| 9 | 13.4 | 92 |
| 10 | 14.9, 15.5 | 89 |
| 11 | 12.4 | 90 |
| 12 | 16.0, 16.5 | 81 |
| 13 | 8.1, 8.5* | 89 |
| 14 | 12.8 | 71 |

| | | |
|---|---|---|
| * Data from Method 2; the others are from Method 1. | | |

The values reported in Table 1 were obtained using HPLC Methods 1 or 2. One retention time is shown for most of these examples. The two isomers that comprise these radiopharmaceuticals are usually not completely resolved by these HPLC methods. Typically there is a shoulder on the main peak reported.

### Analytical Methods

### HPLC Method 1

Column: Vydac C₁₈ (4.6 mm x 25 cm)
Flow rate: 1 mL/min
Solvent A = 0.01 M pH 6 phosphate buffer
Solvent B = acetonitrile
Gradient:
   t = 0 min 100% A
   t = 15 min 70% A, 30% B
   t = 25 min 25% A, 75% B
   Detection by sodium iodide probe

### HPLC Method 2

Column: Zorbax Rx C₁₈ (4.6 mm x 25 cm)
Flow rate: 1 mL/min
Solvent A = 90:10 0.025 M pH 8 phosphate buffer :acetonitrile
Solvent B = 50:50 0.025 M pH 8 phosphate buffer :acetonitrile
Gradient:
   t = 0 min 100% A
   t = 25 min 100% B
   Detection by sodium iodide probe

### Utility

The radiopharmaceuticals provided herein are useful as imaging agents for the diagnosis of cardiovascular disorders, such as thromboembolic disease or atherosclerosis, infectious disease and cancer. The radiopharmaceuticals are comprised of technetium-99m labeled hydrazino or diazenido modified biologically active molecules that selectively localize at sites of disease and thus allow an image to be obtained of the loci using gamma scintigraphy.

Canine Deep Vein Thrombosis Model: This model incorporates the triad of events (hypercoagulatible state, period of stasis, low shear environment) essential for the formation of a venous fibrin-rich actively growing thrombus. The procedure was as follows: Adult mongrel dogs of either sex (9-13 kg) were anesthetized with pentobarbital sodium (35 mg/kg,i.v.) and ventilated with room air via an endotracheal tube (12 strokes/min, 25 ml/kg). For arterial pressure determination, the right femoral artery was cannulated with a saline-filled polyethylene catheter (PE-240) and connected to a Statham pressure transducer (P23ID; Oxnard,CA). Mean arterial blood pressure was determined via damping the pulsatile pressure signal. Heart rate was monitored using a cardiotachometer (Biotach, Grass Quincy, MA) triggered from a lead II electrocardiogram generated by limb leads. The right femoral vein was cannulated (PE-240) for drug administration. A 5 cm segment of both jugular veins was isolated, freed from fascia and circumscribed with silk suture. A microthermister probe was placed on the vessel which serves as an indirect measure of venous flow. A balloon embolectomy catheter was utilized to induce the 15 min period of stasis during which time a hypercoagulatible state was then induced using 5 U thrombin (American Diagnosticia, Greenwich CT) administered into the occluded segment. Fifteen minutes later, flow was reestablished by deflating the balloon. The radiopharmaceutical was infused during the first 5 minutes of reflow and the rate of incorporation monitored using gamma scintigraphy.

Canine Arteriovenous Shunt Model: Adult mongrel dogs of either sex (9-13kg) were anesthetized with pentobarbital sodium (35 mg/kg,i.v.) and ventilated with room air via an endotracheal tube (12 strokes/min, 25 ml/kg). For arterial pressure determination, the left carotid artery was cannulated with a saline-filled polyethylene catheter (PE-240) and connected to a Statham pressure transducer (P23ID; Oxnard,CA). Mean arterial blood pressure was determined via damping the pulsatile pressure signal. Heart rate was monitored using a cardiotachometer (Biotach, Grass Quincy, MA) triggered from a lead II electrocardiogram generated by limb leads. A jugular vein was cannulated (PE-240) for drug administration. The both femoral arteries and femoral veins were cannulated with silicon treated (Sigmacote, Sigma Chemical Co. St Louis, MO), saline filled polyethylene tubing (PE-200) and connected with a 5 cm section of silicon treated tubing (PE-240) to form an extracorporeal arterio-venous shunts (A-V). Shunt patency was monitored using a doppler flow system (model VF-1, Crystal Biotech Inc, Hopkinton, MA) and flow probe (2-2.3 mm, Titronics Med. Inst., Iowa City, IA) placed proximal to the locus of the shunt. All parameters were monitored continuously on a polygraph recorder (model 7D Grass) at a paper speed of 10 mm/min or 25 mm/sec.

On completion of a 15 min post surgical stabilization period, an occlusive thrombus was formed by the introduction of a thrombogenic surface ( 4-0 braided silk thread, 5 cm in length, Ethicon Inc., Somerville, NJ) into the shunt one shunt with the other serving as a control. Two consecutive lhr shunt periods were employed with the test agent administered as an infusion over 5 min beginning 5 min before insertion of the thrombogenic surface. At the end of each 1 hr shunt period the silk was carefully removed and weighed and the % incorporation determined via well counting. Thrombus weight was calculated by subtracting the weight of the silk prior to placement from the total weight of the silk on removal from the shunt. Arterial blood was withdrawn prior to the first shunt and every 30 min thereafter for determination of blood clearance, whole blood collagen-induced platelet aggregation, thrombin-induced platelet degranulation (platelet ATP release), prothrombin time and platelet count. Template bleeding time was also performed at 30 min intervals.

Complexes in which the biologically active molecules, Q, are chemotactic peptides can be evaluated for potential clinical utility as radiopharmaceuticals for the diagnosis of infection by performing imaging studies in a guinea pig model of focal infection.

Guinea Pig Focal Infection Model: Hartley guinea pigs; unspecified sex; weight between 200-250 grams are fasted overnight prior to the procedure. Each guinea pig is anesthetized with a mixture of ketamine 25-55 mg/kg//IM and xylazine 2-5 mg/kg/IM. A #10 trochar needle is used to introduce a 2 inch piece of umbilical string that has been immersed in a 6% sodium caseinate solution (this is the chemoattractant) into the right flank and is placed on the left side of the peritoneal cavity. The placement of the chemoattractant serves as a focal site for white blood cell recruitment. The puncture site is sealed with Nexabain, a skin glue (if required). The animals are allowed to recover for 18 hrs.

Eighteen hours later the guinea pigs are anesthetized with kettamine 25-55 mg/kg//IM and xylazine 2-5 mg/kg/IM to achieve Stage III/Plane III of anesthesia and insure proper injection of the test agent into the lateral saphenous vein . Once the test agent is administered the guinea pigs are placed behind a lead shield and monitored for 1-4 hours. At the appropriate time postinjection, the animals are euthanized with pentobarbital sodium 65 mg/kg, I.V.. and a biodistribution performed. Throughout the course of the study, blood samples are withdrawn via cardiac puncture.

### Results

The results of the evaluation of the radiopharmaceutical of Example 1 in the Canine Arteriovenous Shunt model is shown in Figure 1. Included are results for the positive control, In-111-labeled autologous platelets and the negative controls, Tc-99m-albumin and I-125-fibrinogen. All values are for the first 1 hour shunt period. The data show that the radiopharmaceutical of Example 1 is taken up in thrombi under both mixed arterial and venous conditions equivalently to the positive control and to a significantly greater extent than the negative controls. The thrombus-to-blood ratios (target-to-background ratios), 114 (mixed arterial) and 3.1 (venous) indicate that the thrombi can be readily differentiated from the surrounding tissue and fluid by standard imaging techniques familiar to those skilled in the art. The important advantage of the radiopharmaceutical of Example 1 over the positive control, In-111-platelets, is that the laborious extracorporeal labeling procedure used to label autologous platelets with In-111 is not necessary. This shortens the time needed to obtain diagnostic information and avoids the potential risk of exposure of the nuclear medicine practitioner to blood -borne pathogens, such as HIV.

## Claims

1. A radiopharmaceutical of formula:
**[(Q)**_{**d'**}**L**_{**n**}**-C**_{**h'**}**]**_{**x**}**-M**_{**t**}**(A**_{**L1**}**)**_{**y**}**(A**_{**L2**}**)**_{**z**} (1)
and pharmaceutically acceptable salts thereof wherein,
Q is a biologically active molecule selected from the group: IIb/IIIa receptor antagonists, IIb/IIIa receptor ligands, fibrin binding peptides, leukocyte binding peptides, chemotactic peptides, somatostatin analogs, and selectin binding peptides;
d' is 1 to 3;
Lₙ is:
- (CR⁵⁵R⁵⁶)_{g"}-[Y¹(CR⁵⁵R⁵⁶)_{f}Y²]_{f'}-(CR⁵⁵R⁵⁶)_{g"}-,
wherein:
g" is 0-5;
f is 0-5;
f' is 1-5;
Y¹ and Y², at each occurrence, are independently selected from: O, NR⁵⁶, C=O, C(=O)O, OC(=O)O, C(=O)NH, C=NR⁵⁶, S, SO, SO₂, SO₃, NHC(=O), (NH)₂C(=O), (NH)₂C=S;
R⁵⁵ and R⁵⁶ are independently selected at each occurrence from:
hydrogen, C₁-C₁₀ alkyl and alkaryl;
x and y are 1;
Z is 1 or 2;
Mₜ is ^{99m}Tc;
C_{h'} is selected from the group: R⁴⁰N=N⁺= and R⁴⁰R⁴¹N-N=; wherein
R⁴⁰ is independently selected at each occurrence from the group: aryl substituted with 0-3 R⁵², and heterocycle substituted with 0-3 R⁵²;
R⁴¹ is independently selected from the group: hydrogen, aryl substituted with 0-1 R⁵², C₁-C₃ alkyl substituted with 0-1 R⁵², and
a heterocycle substituted with 0-1 R⁵²;
R⁵² is independently selected at each occurrence from the group: a bond to Lₙ, -CO₂R⁵³, -CH₂OR⁵³, -SO₃H, -SO₂R^{53a}, -N(R⁵³)₂, -N(R⁵³)₃+ -NHC(=NH)NHR⁵³, and -OCH₂CO₂H; R⁵³ and R^{53a} are each independently selected at each occurrence from the group: hydrogen and C₁-C₃ alkyl;
A_{L1} is a functionalized aminocarboxylate;
A_{L2} is an ancillary ligand capable of stabilizing the radiopharmaceutical selected from the group: 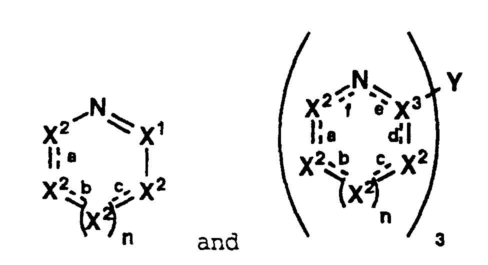 wherein
n is 0
X¹ is independently selected at each occurrence from the group: CR⁶⁴ and N;
X² is independently selected at each occurrence from the group: CR⁶⁴, CR⁶⁴R⁶⁴, N, NR⁶⁴, O and S;
X³ is independently selected at each occurrence from the group: C, CR⁶⁴, and N;
provided the total number of heteroatoms in each ring of the ligand A_{L2} is 1 to 4;
Y is BR⁶⁴⁻;
and a, b, c, d, e and f indicate the positions of optional double bonds, provided that one of e and f is a double bond.
R⁶⁴ is independently selected at each occurrence from the group:
H, C₁-C₃ alkyl substituted with 0-3 R⁶⁵, aryl substituted with 0-3 R⁶⁵, and R⁶⁵;
or, alternatively, two R⁶⁴ may be taken together with the atom or atoms to which they are attached to form a fused aromatic or heterocyclic ring, substituted with 0-3 R⁶⁵;
R⁶⁵ is independently selected at each occurrence from the group: -NO₂, -CO₂R⁶⁶, -OR⁶⁶, -SO₃H, and -OCH₂CO₂H; and
R⁶⁶ is hydrogen.

2. The radiopharmaceutical of Claim 1 wherein:
Q is a biologically active molecule selected from the group: IIb/IIIa receptor antagonists and chemotactic peptides;
d' is 1;
Y¹ and Y², at each occurrence, are independently selected from:
O, NR⁵⁶, C=O, C(=O)O, OC(=O)O, C(=O)NH, C=NR⁵⁶, NHC(=O), (NH)₂C(=O);
R⁵⁵ and R⁵⁶ are hydrogen;
z is 1;
R⁴⁰ is heterocycle substituted with R⁵²;
R⁴¹ is hydrogen;
R⁵² is a bond to Lₙ;
A_{L1} is tricine;
X² is independently selected at each occurrence from the group: CR⁶⁴, CR⁶⁴R⁶⁴, N, NR⁶⁴ and O;
X³ is N;
provided the total number of heteroatoms in each ring of the ligand A_{L2} is 2 to 3;
R⁶⁴ is independently selected at each occurrence from the group:
H, C₁-C₃ alkyl substituted with 0-1 R⁶⁵, aryl substituted with 0-1 R⁶⁵; and R⁶⁵;
R⁶⁵ is independently selected at each occurrence from the group: -NO₂, -CO₂R⁶⁶, -OR⁶⁶, and -SO₃H.

3. The radiopharmaceutical of Claim 1 wherein:
Q is 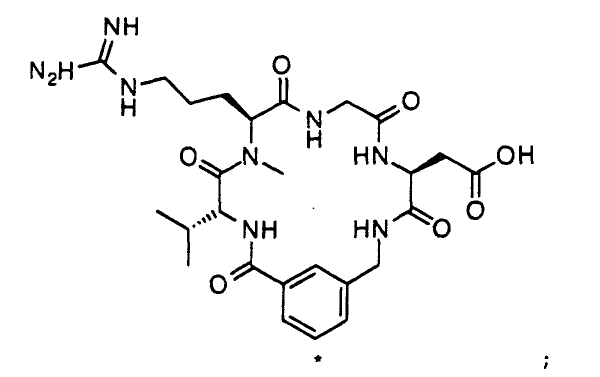
d' is 1;
Lₙ is attached to Q at the carbon atom designated with a * and has the formula:
- (C=O)NH(CH₂)₅C(=O)NH-;
C_{h'} is 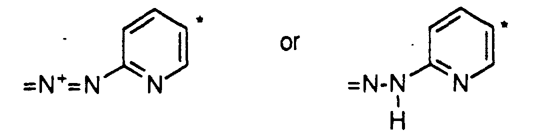 and is attached to Ln at the carbon atom designated with a *;
Mₜ is ^{99m}Tc;
A_{L1} is tricine;
x, y and z are 1;
and A_{L2} is selected from the group: 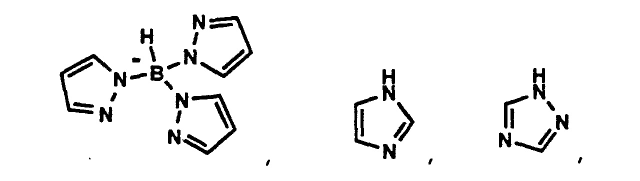 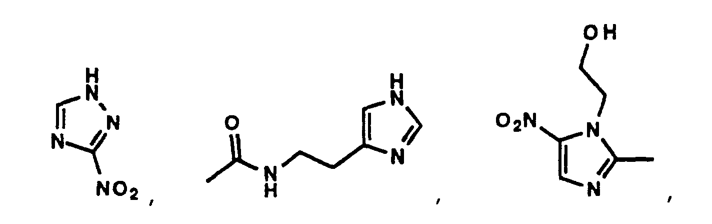 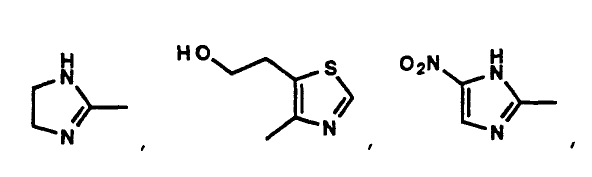 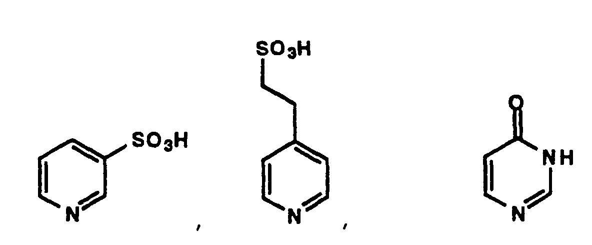 and

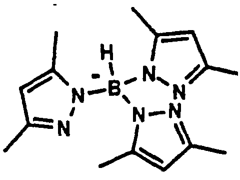

4. A compound according to any one of Claim 1-3 for use in therapy.

5. Use of radiopharmaceutical according to any one of claims 1-3 for the preparation of a medicament for use in radioimaging a mammal.

6. Use of radiopharmaceutical according to any one of claims 1-3 for the preparation of a medicament for use in visualizing sites of platelet deposition in a mammal by radioimaging.

7. Use of radiopharmaceutical according to any one of claims 1-3 for the preparation of a medicament for use in determining platelet deposition in a mammal.

8. Use of radiopharmaceutical according to any one of claims 1-3 for the preparation of a medicament for use in diagnosing a disorder associated with platelet deposition in a mammal.

9. A kit for preparing a radiopharmaceutical comprising:
(a) a predetermined quantity of a sterile, pharmaceutically acceptable reagent of formula:
**(Q) d'L**_{**n**}**-C**_{**h**}**;**
(b) a predetermined quantity of a sterile, pharmaceutically acceptable first ancillary ligand, A_{L1}, which is a functionalized aminocarboxylate;
(c) a predetermined quantity of a sterile, pharmaceutically acceptable second ancillary ligand, A_{L2}, selected from the group: 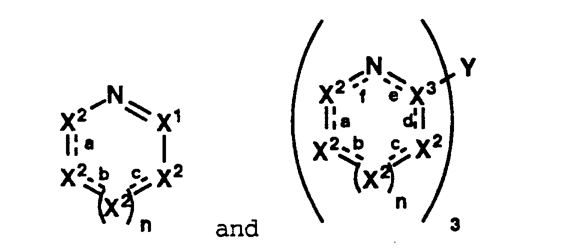
(d) a predetermined quantity of a sterile, pharmaceutically acceptable reducing agent; and
(e) optionally, a predetermined quantity of one or more sterile, pharmaceutically acceptable components selected from the group: transfer ligands, buffers, lyophilization aids, stabilization aids, solubilization aids and bacteriostats;
wherein:
Q, d', Lₙ, X¹, X², X³, n, y, a, b, c, d, e and f are as defined in Claim 1;
Cₕ is a radionuclide metal chelator selected from the group: R⁴⁰R⁴¹N-N=C(C₁-C₃ alkyl)₂ and R⁴⁰NNH₂-, and R⁴⁰R⁴¹N-N=CR⁸⁰R⁸¹, wherein,
R⁴⁰ is independently selected at each occurrence from the group: a bond to Lₙ, C₁-C₁₀ alkyl substituted with 0-3 R⁵², aryl substituted with 0-3 R⁵², cycloaklyl substituted with 0-3 R⁵², heterocycle substituted with 0-3 R⁵², heterocycloalkyl substituted with 0-3 R⁵², aralkyl substituted with 0-3 R⁵² and alkaryl substituted with 0-3 R⁵²;
R⁴¹ is independently selected from the group: hydrogen, aryl substituted with 0-3 R⁵², C₁-C₁₀ alkyl substituted with 0-3 R⁵², and a heterocycle substituted with 0-3 R⁵²;
R⁵² is independently selected at each occurrence from the group: a bond to Lₙ, =O, F, Cl, Br, I, -CF₃, -CN, -CO₂R⁵³, -C(=O)R⁵³, -C(=O)N(R⁵³)₂, -CHO, -CH₂OR⁵³, -OC(=O)R⁵³, -OC(=O)OR^{53a}, -OR⁵³, -OC(=O)N(R⁵³)₂, -NR⁵³C(=O)R⁵³, -NR⁵⁴C(=O)OR^{53a}, -N(R⁵³)₃⁺, -NR⁵³C(=O)N(R⁵³)₂, -NR⁵⁴SO₂N(R⁵³)₂, -NR⁵⁴SO₂R^{53a}, -SO₃H, -SO₂R^{53a}, -SR⁵³, -S(=O)R^{53a}, -SO₂N(R⁵³)₂, -N(R⁵³)₂, -NHC(=NH)NHR⁵³, -C(=NH)NHR⁵³, =NOR5³, NO₂, -C(=O)NHOR⁵³, -C(=O)NHNR⁵³R^{53a}, -OCH₂CO₂H, 2-(1-morpholino)ethoxy;
R⁵³, R^{53a}, and R⁵⁴ are each independently selected at each occurrence from the group: hydrogen, C₁-C₆ alkyl, and a bond to Lₙ;
R⁸⁰ and R⁸¹ are independently selected from the group:
H, C₁-C₁₀ alkyl, -CN, -CO₂R⁸⁵, -C(=O)R⁸⁵, -C(=O)N(R⁸⁵)₂, C₂- C₁₀ 1-alkene substituted with 0-3 R⁸⁴, C₂ - C₁₀ 1-alkyne substituted with 0-3 R⁸⁴, aryl substituted with 0-3 R⁸⁴, unsaturated heterocycle substituted with 0-3 R⁸⁴, and unsaturated carbocycle substituted with 0-3 R⁸⁴, provided that when one of R⁸⁰ and R⁸¹ is H or alkyl, then the other is not H or alkyl;
or, alternatively, R⁸⁰ and R⁸¹, may be taken together with the shown divalent carbon radical to form: 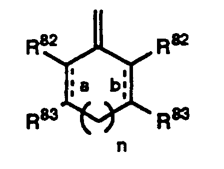 wherein:
R⁸² and R⁸³ may be independently selected from the group: H, R⁸⁴, C₁-C₁₀ alkyl substituted with 0-3 R⁸⁴, C₂-C₁₀ alkenyl substituted with 0-3 R⁸⁴, C₂-C₁₀ alkynyl substituted with 0-3 R^{84,} aryl substituted with 0-3 R^{84,} heterocycle substituted with 0-3 R⁸⁴ and carbocycle substituted with 0-3 R⁸⁴;
or, alternatively, R⁸², R⁸³ may be taken together to form a fused aromatic or heterocyclic ring;
R⁸⁴ is independently selected at each occurrence from the group: =O, F, Cl, Br, I, -CF₃, -CN, -CO₂R⁸⁵, -C(=O)R⁸⁵, -C(=O)N(R⁸⁵)₂, - N(R⁸⁵)₃+ -CH₂OR⁸⁵, -OC(=O)R⁸⁵, -OC(=C)OR^{85a}, -OR⁸⁵, -OC(=O)N(R⁸⁵)₂, -NR⁸⁵C(=O)R⁸⁵, -NR⁸⁶C(=O)OR^{85a}, -NR⁸⁵C(=O)N(R⁸⁵)₂, -NR⁸⁶SO₂N(R⁸⁵)₂, -NR⁸⁶SO₂R^{85a}, -SO₃H, -SO₂R^{85a}, -SR⁸⁵, -S(=O)R^{85a}, -SO₂N(R⁸⁵)₂, -N(R⁸⁵)₂, -NHC(=NH)NHR⁸⁵, -C(=NH)NHR⁸⁵, =NOR⁸⁵, -C(=O)NHOR⁸⁵, -OCH₂CO₂H, 2-(1-morpholino)ethoxy; and
R⁸⁵, R^{85a}, and R⁸⁶ are each independently selected at each occurrence from the group: hydrogen and C₁-C₆ alkyl.

10. The kit of Claim 9, wherein:
A_{L1} is a tricine;
Q is a biologically active molecule selected from the group: IIb/IIIa receptor antagonists, and chemotactic peptides;
d' is 1;
Y¹ and Y², at each occurrence, are independently selected from:
O, NR⁵⁶, C=O, C(=O)O, OC(=O)O, C(=O)NH, C=NR⁵⁶, NHC(=O), (NH)₂C(=O);
R⁵⁵ and R⁵⁶ are hydrogen;
R⁴⁰ is heterocycle substituted with R⁵²;
R⁴¹ is hydrogen;
R⁵² is a bond to Lₙ;
R⁸⁰ is independently selected at each occurrence from the group: -CO₂R⁸⁵' C₂ - C₃ 1-alkene substituted with 0-1 R⁸⁴' aryl substituted with 0-1 R⁸⁴' and unsaturated heterocycle substituted with 0-1 R⁸⁴;
R⁸¹ is H;
R⁸⁴ is independently selected at each occurrence from the group: -CO₂R⁸⁵, -OR⁸⁵, -SO₃H, and -N(R⁸⁵)₂;
R⁸⁵ is independently selected at each occurrence from the group: hydrogen and methyl;
X¹ is independently selected at each occurrence from the group: CR⁶⁴ and N;
X² is independently selected at each occurrence from the group: CR⁶⁴, CR⁶⁴R⁶⁴, N, NR⁶⁴, and O;
X³ is N;
Y is BR⁶⁴⁻;
provided the total number of heteroatoms in each ring of the ligand A_{L2} is 2 to 3;
R⁶⁴ is independently selected at each occurrence from the group:
H, C₁-C₃ alkyl substituted with 0-1 R^{65,} aryl substituted with 0-1 R⁶⁵, and R⁶⁵;
R⁶⁵ is independently selected at each occurrence from the group: -NO₂, -CO₂R⁶⁶, -OR⁶⁶, and -SO₃H;
R⁶⁶ is hydrogen;
n is 0;
and a, b, c, d, e and f indicate the positions of optional double bonds, provided that one of e and f is a double bond.

11. The kit of Claim 9, wherein:
A_{L2} is selected from the group: 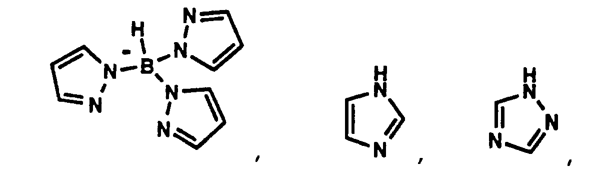 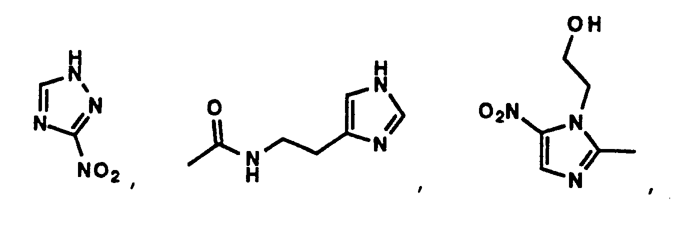 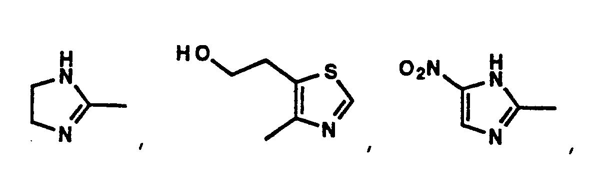 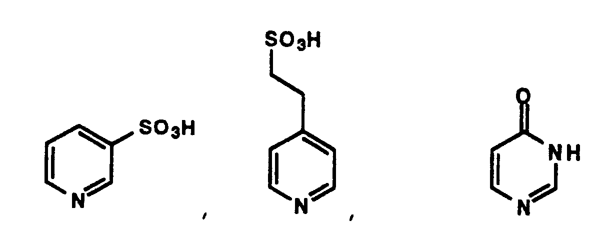 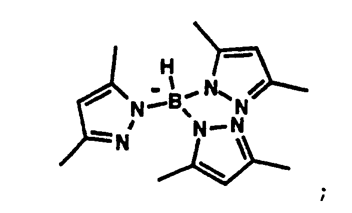
Q is 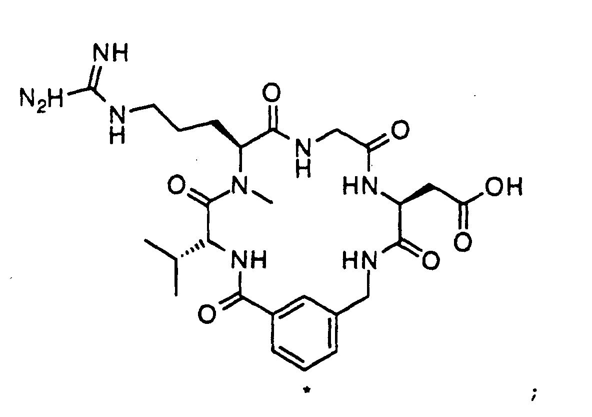
d' is 1;
Lₙ is attached to Q at the carbon atom designated with a * and has the formula:
- (C=O)NH(CH₂)₅C(=O)NH-;
Cₕ is selected from R⁴⁰NNH₂- and R⁴⁰R⁴¹N-N=CR⁸⁰R⁸¹, wherein,
R⁴⁰ is independently selected at each occurrence from the group: heterocycle substituted with R⁵²;
R⁴¹ is hydrogen;
R⁵² is a bond to Lₙ;
R⁸⁰ is independently selected at each occurrence from the group: -CO₂R⁸⁵, C₂ - C₃ 1-alkene substituted with 0-1 R^{84,} aryl substituted with 0-1 R⁸⁴ and unsaturated heterocycle substituted with 0-1 R⁸⁴;
R⁸¹ is H;
R⁸⁴ is independently selected at each occurrence from the group: -CO₂R⁸⁵, -OR⁸⁵, -SO₃H, and -N(R⁸⁵)₂;
R⁸⁵ is independently selected at each occurrence from the group: hydrogen and methyl.

## Patentansprüche

1. Radiopharmazeutikum der Formel:
[(Q)_{d'}Lₙ-C_{h'}]ₓ-Mₜ(A_{L1})_{y}(A_{L2})_{z} (1)
und dessen pharmazeutisch verträgliche Salze, wobei
Q ein biologisch aktives Molekül ist, ausgewählt aus: IIb/IIIa Rezeptorantagonisten, IIb/IIIa Rezeptorliganden, Fibrin-bindenden Peptiden, Leukozyten-bindenden Peptiden, chemotaktischen Peptiden, Somatostatinanaloga und Selectin-bindenden Peptiden;
d' 1 bis 3 ist;
Lₙ
-(CR⁵⁵R⁵⁶)_{g"}-[Y¹(CR⁵⁵R⁵⁶)_{f}Y²]_{f'}-(CR⁵⁵R⁵⁶)_{g"}-
ist, wobei:
g" h 0 bis 5 ist;
f 0 bis 5 ist;
f' 1 bis 5 ist;
Y¹ und Y² jeweils unabhängig ausgewählt sind aus: O, NR⁵⁶, C=O, C(=O)O, OC(=O)O, C(=O)NH, C=NR⁵⁶, S, SO, SO₂, SO₃, NHC(=O), (NH)₂C(=O), (NH)₂C=S;
R⁵⁵ und R⁵⁶ jeweils unabhängig ausgewählt sind aus:
Wasserstoff, C₁-C₁₀-Alkyl und Alkaryl;
x und y 1 sind;
Z 1 oder 2 ist;
Mₜ ^{99m}Tc ist;
Cₕ ausgewählt ist aus: R⁴⁰N=N⁺= und R⁴⁰R⁴¹N-N=; wobei
R⁴⁰ jeweils unabhängig ausgewählt ist aus: Aryl, substituiert mit 0 bis 3 R⁵², und Heterocyclus, substituiert mit 0 bis 3 R⁵²;
R⁴¹ unabhängig ausgewählt ist aus: Wasserstoff, Aryl, substituiert mit 0 bis 1 R⁵², C₁-C₃-Alkyl, substituiert mit 0 bis 1 R⁵² und einem Heterocyclus, substituiert mit 0 bis 1 R⁵²;
R⁵² jeweils unabhängig ausgewählt ist aus: einer Bindung zu Lₙ, -CO₂R⁵³, -CH₂OR⁵³, -SO₃H, -SO₂R^{53a}, -N(R⁵³)₂, -N(R⁵³)₃₊, -NHC(=NH)NHR⁵³ und -OCH₂CO₂H;
R⁵³ und R^{53a} jeweils unabhängig ausgewählt sind aus: Wasserstoff und C₁-C₃-Alkyl;
A_{L1} ein funktionalisiertes Aminocarboxylat ist;
A_{L2} ein Hilfsligand zur Stabilisierung des Radiopharmazeutikums ist, ausgewählt aus: 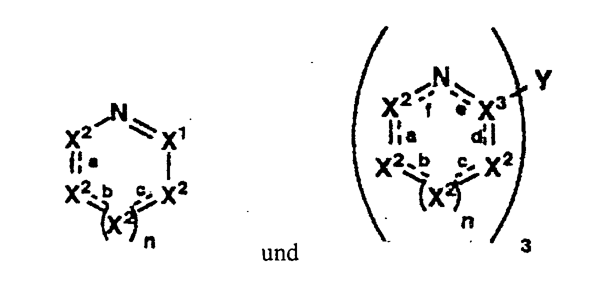 wobei
n 0 ist;
X¹ jeweils unabhängig ausgewählt ist aus: CR⁶⁴ und N;
X² jeweils unabhängig ausgewählt ist aus: CR⁶⁴, CR⁶⁴R⁶⁴, N, NR⁶⁴, O und S;
X³ jeweils unabhängig ausgewählt ist aus: C, CR⁶⁴ und N;
mit der Maßgabe, dass die Gesamtzahl von Heteroatomen in jedem Ring des Liganden A_{L2} 1 bis 4 ist;
Y BR⁶⁴⁻ ist;
und a, b, c, d, e und f die Positionen optionaler Doppelbindungen angeben, mit der Maßgabe, dass eines von e und f eine Doppelbindung ist;
R⁶⁴ jeweils unabhängig ausgewählt ist aus:
H, C₁-C₃-Alkyl, substituiert mit 0 bis 3 R⁶⁵, Aryl, substituiert mit 0 bis 3 R⁶⁵, und R⁶⁵;
oder, alternativ, zwei R⁶⁴ zusammen mit dem Atom oder den Atomen, an welche sie gebunden sind, einen kondensierten aromatischen oder heterocyclischen Ring, substituiert mit 0 bis 3 R⁶⁵, bilden können;
R⁶⁵ jeweils unabhängig ausgewählt ist aus: -NO₂, -CO₂R⁶⁶, -OR⁶⁶, -SO₃H, und -OCH₂CO₂H; und
R⁶⁶ Wasserstoff ist.

2. Radiopharmazeutikum nach Anspruch 1, wobei:
Q ein biologisch aktives Molekül ist, ausgewählt aus: IIb/IIIa Rezeptorantagonisten und chemotaktischen Peptiden;
d' 1 ist;
Y¹ und Y² jeweils unabhängig ausgewählt sind aus:
O, NR⁵⁶, C=O, C(=O)O, OC(=O)O, C(=O)NH, C=NR⁵⁶, NHC(=O), (NH)₂C(=O);
R⁵⁵ und R⁵⁶ Wasserstoff sind;
Z 1 ist;
R⁴⁰ ein Heterocyclus, substituiert mit R⁵², ist;
R⁴¹ Wasserstoff ist;
R⁵² eine Bindung zu Lₙ ist;
A_{L1} Tricin ist;
X² jeweils unabhängig ausgewählt ist aus: CR⁶⁴, CR⁶⁴R⁶⁴, N, NR⁶⁴ und O;
X³ N ist;
mit der Maßgabe, dass die Gesamtzahl von Heteroatomen in jedem Ring des Liganden A_{L2} 2 bis 3 ist;
R⁶⁴ jeweils unabhängig ausgewählt ist aus:
H, C₁-C₃-Alkyl, substituiert mit 0 bis 1 R⁶⁵, Aryl, substituiert mit 0 bis 1 R⁶⁵; und R⁶⁵;
R⁶⁵ jeweils unabhängig ausgewählt ist aus: -NO₂, -CO₂R⁶⁶, -OR⁶⁶, und -SO₃H.

3. Radiopharmazeutikum nach Anspruch 1, wobei:
Q 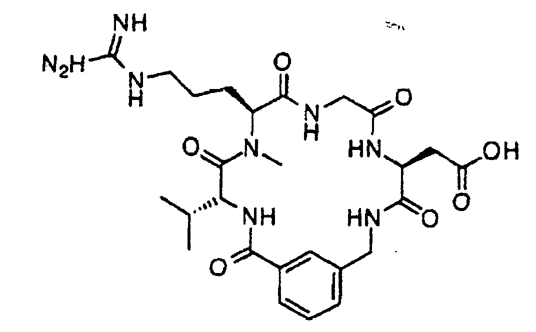 ist;
d' 1 ist;
Lₙ an dem mit einem * gekennzeichneten Kohlenstoffatom mit Q verbunden ist und die Formel
-(C=O)NH(CH₂)₅C(=O)NH-
aufweist;
C_{h'} 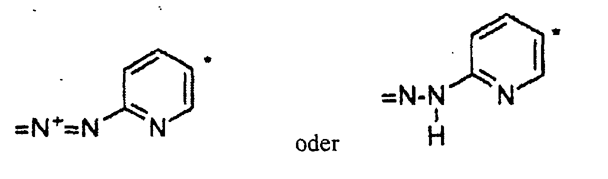 ist und an dem mit einem * gekennzeichneten Kohlenstoffatom mit Lₙ verbunden ist;
Mₜ ^{99m}Tc ist;
A_{L1} Tricin ist;
x, y und z 1 sind;
und A_{L2} ausgewählt ist aus: 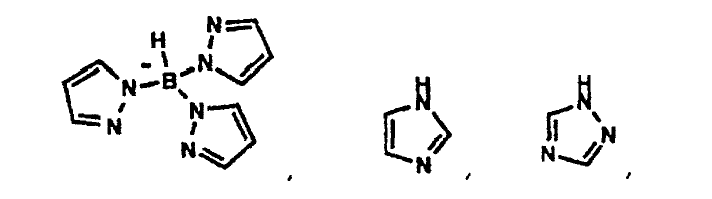 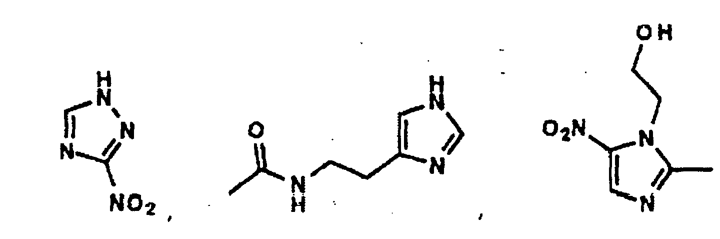 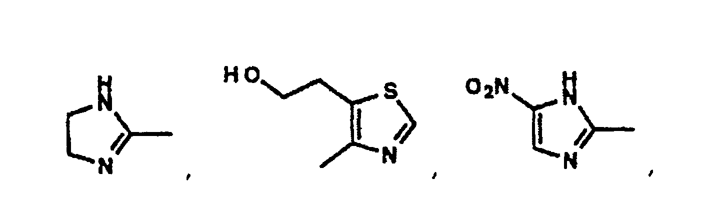 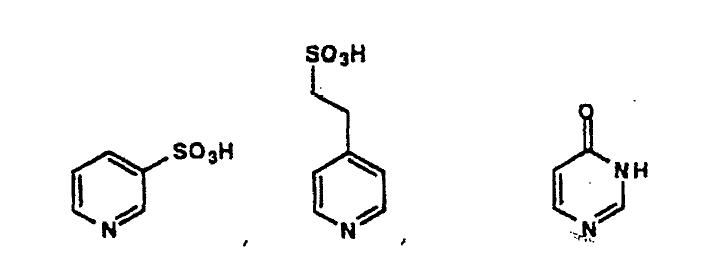 und

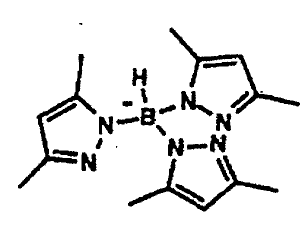

4. Verbindung gemäß einem der Ansprüche 1 bis 3 zur Verwendung in der Therapie.

5. Verwendung des Radiopharmazeutikums gemäß einem der Ansprüche 1 bis 3 für die Herstellung eines Medikaments zur Verwendung in der Szintigraphie eines Säugers.

6. Verwendung des Radiopharmazeutikums gemäß einem der Ansprüche 1 bis 3 für die Herstellung eines Medikaments zur Verwendung beim Visualisieren von Stellen von Thrombozyten-Ablagerungen in Säugern durch Szintigraphie.

7. Verwendung des Radiopharmazeutikums gemäß einem der Ansprüche 1 bis 3 für die Herstellung eines Medikaments zur Verwendung beim Bestimmen von Thrombozyten-Ablagerungen in einem Säuger.

8. Verwendung des Radiopharmazeutikums gemäß einem der Ansprüche 1 bis 3 für die Herstellung eines Medikaments zur Verwendung beim Diagnostizieren einer Erkrankung in Verbindung mit Thrombozyten-Ablagerung in einem Säuger.

9. Kit zur Herstellung eines Radiopharmazeutikums, umfassend:
(a) eine bestimmte Menge eines sterilen, pharmazeutisch verträglichen Reagens der Formel:
(Q)_{d'}Lₙ-Cₕ;
(b) eine bestimmte Menge eines sterilen, pharmazeutisch verträglichen ersten Hilfsliganden, A_{L1}, der ein funktionalisiertes Aminocarboxylat ist;
(c) eine bestimmte Menge eines sterilen, pharmazeutisch verträglichen zweiten Hilfsliganden, A_{L2}, ausgewählt aus: 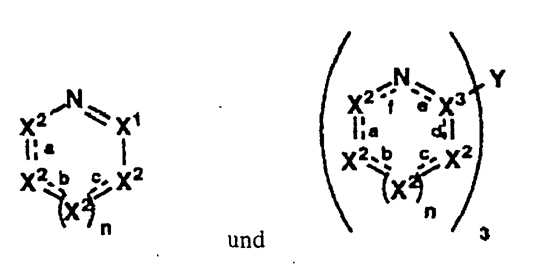
(d) eine bestimmte Menge eines sterilen, pharmazeutisch verträglichen Reduktionsmittels; und
(e) gegebenenfalls, eine bestimmte Menge einer oder mehrerer steriler, pharmazeutisch verträglicher Komponenten, ausgewählt aus: Transferliganden, Puffer, Gefriertrocknungs-Hilfsmitteln, Stabilisierungshilfen, Lösungshilfen und Bakteriostatika;
wobei:
Q, d', Lₙ, X¹, X², X³, n, Y, a, b, c, d, e und f wie in Anspruch 1 definiert sind;
Cₕ ein Radionuklid-Metallchelator ist, ausgewählt aus: R⁴⁰R⁴¹N-N=C(C₁-C₃-Alkyl)₂ und R⁴⁰NNH₂- und R⁴⁰R⁴¹N-N=CR⁸⁰R⁸¹, wobei
R⁴⁰ jeweils unabhängig ausgewählt ist aus: einer Bindung zu Lₙ, C₁-C₁₀-Alkyl, substituiert mit 0 bis 3 R⁵², Aryl, substituiert mit 0 bis 3 R⁵², Cycloalkyl, substituiert mit 0 bis 3 R⁵², Heterocyclus, substituiert mit 0 bis 3 R⁵², Heterocycloalkyl, substituiert mit 0 bis 3 R⁵², Aralkyl substituiert mit 0 bis 3 R⁵² und Alkaryl, substituiert mit 0 bis 3 R⁵²;
R⁴¹ unabhängig ausgewählt ist aus: Wasserstoff, Aryl, substituiert mit 0 bis 3 R⁵², C₁-C₁₀-Alkyl, substituiert mit 0 bis 3 R⁵², und einem Heterozyklus, substituiert mit 0 bis 3 R⁵²;
R⁵² jeweils unabhängig ausgewählt ist aus: einer Bindung zu Lₙ, =O, F, Cl, Br, I, -CF₃, -CN, -CO₂R⁵³, -C(=O)R⁵³, -C(=O)N(R⁵³)₂, -CHO, -CH₂OR⁵³, -OC(=O)R⁵³, -OC(=O)OR^{53a}, -OR⁵³, -OC(=O)N(R⁵³)₂, -NR⁵³C(=O)R⁵³, -NR⁵⁴C(=O)OR^{53a}, -N(R⁵³)₃₊, -NR⁵³C(=O)N(R⁵³)₂, -NR⁵⁴SO₂N(R⁵³)₂, -NR⁵⁴SO₂R^{53a}, -SO₃H, -SO₂R^{53a}, -SR⁵³, -S(=O)R^{53a}, -SO₂N(R⁵³)₂, -N(R⁵³)₂, -NHC(=NH)NHR⁵³, -C(=NH)NHR⁵³, =NOR⁵³, NO2, -C(=O)NHOR⁵³, -C(=O)NHNR⁵³R^{53a}, -OCH₂CO₂H, 2-(1-Morpholino)ethoxy;
R⁵³, R^{53a} und R⁵⁴ jeweils unabhängig ausgewählt sind aus: Wasserstoff, C₁-C₆-Alkyl, und einer Bindung zu Lₙ;
R⁸⁰ und R⁸¹ unabhängig ausgewählt sind aus:
H, C₁-C₁₀-Alkyl, -CN, -CO₂R⁸⁵, -C(=O)R⁸⁵, -C(=O)N(R⁸⁵)₂, C₂-C₁₀-1-Alken, substituiert mit 0 bis 3 R⁸⁴, C₂-C₁₀-1-Alkin, substituiert mit 0 bis 3 R⁸⁴, Aryl, substituiert mit 0 bis 3 R⁸⁴, ungesättigtem Heterocyclus, substituiert mit 0 bis 3 R⁸⁴, ungesättigten Carbocyclus, substituiert mit 0 bis 3 R⁸⁴, mit der Maßgabe, dass, wenn einer von R⁸⁰ und R⁸¹ H oder Alkyl ist, der andere nicht H oder Alkyl ist;
oder, alternativ, R⁸⁰ und R⁸¹ zusammen mit dem dargestellten divalenten Kohlenstoffradikal eine Struktureinheit der Formel 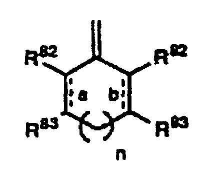 bilden können, wobei:
R⁸² und R⁸³ unabhängig ausgewählt sein können aus: H, R⁸⁴, C₁-C₁₀-Alkyl, substituiert mit 0 bis 3 R⁸⁴, C₂-C₁₀-Alkenyl, substituiert mit 0 bis 3 R⁸⁴, C₂-C₁₀-Alkinyl, substituiert mit 0 bis 3 R⁸⁴, Aryl, substituiert mit 0 bis 3 R⁸⁴,
Heterocyclus, substituiert mit 0 bis 3 R⁸⁴ und Carbocyclus, substituiert mit 0 bis 3 R⁸⁴;
oder, alternativ, R⁸², R⁸³ zusammen einen kondensierten aromatischen oder heterocyclischen Ring ausbilden können;
R⁸⁴ jeweils unabhängig ausgewählt ist, aus: =O, F, Cl, Br, I, -CF₃, -CN, -CO₂R⁸⁵, -C(=O)R⁸⁵, -C(=O)N(R⁸⁵)₂, -N(R⁸⁵)₃₊ -CH₂OR⁸⁵, -OC(=O)R⁸⁵, -OC(=O)OR^{85a}, -OR⁸⁵, -OC(=O)N(R⁸⁵)₂, -NR⁸⁵C(=O)R⁸⁵, -NR⁸⁶C(=O)OR^{85a}, -NR⁸⁵C(=O)N(R⁸⁵)₂, -NR⁸⁶SO₂N(R⁸⁵)₂, -NR⁸⁶SO₂R^{85a}, -SO₃H, -SO₂R^{85a}, -SR⁸⁵, -S(=O)R^{85a}, -SO₂N(R⁸⁵)₂, -N(R⁸⁵)₂, -NHC(=NH)NHR⁸⁵, -C(=NH)NHR⁸⁵, =NOR⁸⁵, -C(=O)NHOR⁸⁵, -OCH₂CO₂H, 2-(1-Morpholino) ethoxy; und
R⁸⁵, R^{85a} und R⁸⁶ jeweils unabhängig ausgewählt sind aus: Wasserstoff und C₁-C₆-Alkyl.

10. Kit nach Anspruch 9, wobei:
A_{L1} Tricin ist;
Q ein biologisch aktives Molekül ist, ausgewählt aus: IIb/IIIa Rezeptor-Antagonisten und chemotaktiscnen Peptiden;
d' 1 ist;
Y¹ und Y² jeweils unabhängig ausgewählt sind aus:
O, NR⁵⁶, C=O, C(=O)O, OC(=O)O, C(=O)NH, C=NR⁵⁶, NHC(=O), (NH)₂C(=O);
R⁵⁵ und R⁵⁶ Wasserstoff sind;
R⁴⁰ ein Heterocyclus, substituiert mit R⁵², ist;
R⁴¹ Wasserstoff ist;
R⁵² eine Bindung zu Lₙ ist;
R⁸⁰ jeweils unabhängig ausgewählt ist aus:
-CO₂R⁸⁵, C₂-C₃-1-Alken, substituiert mit 0 bis 1 R⁸⁴, Aryl, substituiert mit 0 bis 1 R⁸⁴, und ungesättigtem Heterocyclus, substituiert mit 0 bis 1 R⁸⁴;
R⁸¹ H ist;
R⁸⁴ jeweils unabhängig ausgewählt ist aus: -CO₂R⁸⁵, -OR⁸⁵, -SO₃H und -N(R⁸⁵)₂;
R⁸⁵ jeweils unabhängig ausgewählt ist aus: Wasserstoff und Methyl;
X¹ jeweils unabhängig ausgewählt ist aus: CR⁶⁴ und N;
X² jeweils unabhängig ausgewählt ist aus: CR⁶⁴, CR⁶⁴R⁶⁴, N, NR⁶⁴ und O;
X³ N ist;
Y BR⁶⁴⁻ ist;
mit der Maßgabe, dass die Gesamtzahl von Heteroatomen in jedem Ring des Liganden A_{L2} 2 bis 3 ist;
R⁶⁴ jeweils unabhängig ausgewählt ist aus:
H, C₁-C₃-Alkyl, substituiert mit 0 bis 1 R⁶⁵, Aryl, substituiert mit 0 bis 1 R⁶⁵, und R⁶⁵;
R⁶⁵ jeweils unabhängig ausgewählt ist aus: -NO₂, -CO₂R⁶⁶, -OR⁶⁶ und -SO₃H;
R⁶⁶ Wasserstoff ist;
n gleich 0 ist
und a, b, c, d, e und f die Positionen optionaler Doppelbindungen angeben, mit der Maßgabe, dass eines von e und f eine Doppelbindung ist.

11. Kit gemäß Anspruch 9, wobei:
A_{L2} ausgewählt ist, aus: 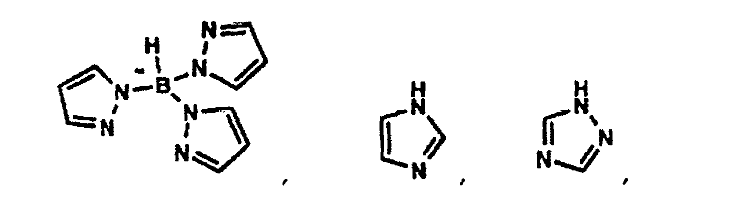 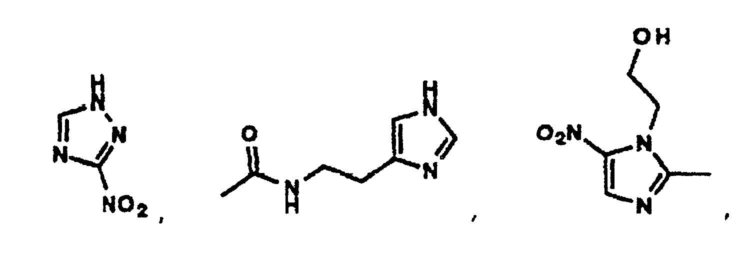 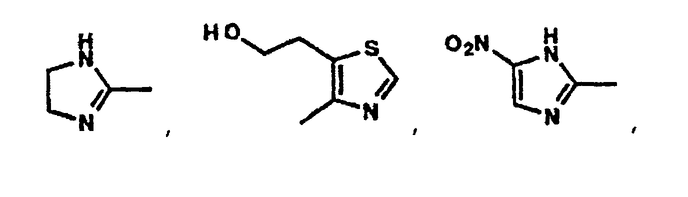 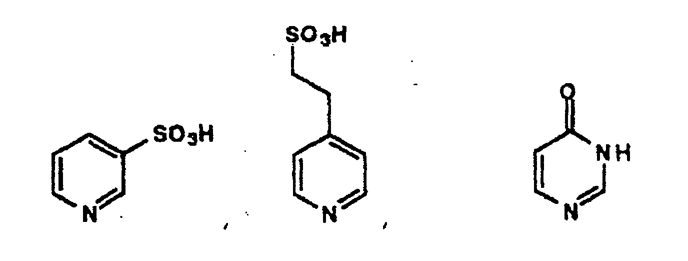 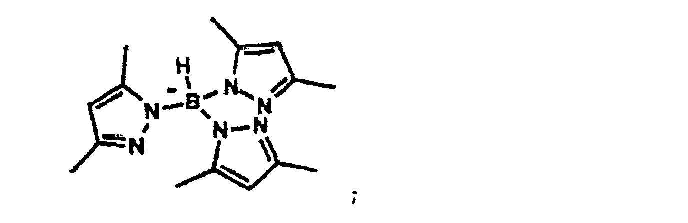
Q 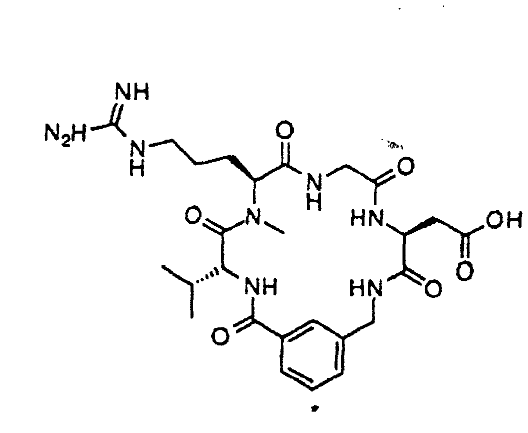 ist;
d' 1 ist;
Lₙ an dem mit einem * gekennzeichneten Kohlenstoffatom mit Q verbunden ist und die Formel
-(C=O)NH(CH₂)₅C(=O)NH-
aufweist;
Cₕ ausgewählt ist aus: R⁴⁰NNH₂- und R⁴⁰R⁴¹N-N=CR⁸⁰R⁸¹, wobei
R⁴⁰ jeweils unabhängig ausgewählt ist aus: Heterocyclus, substituiert mit R⁵²;
R⁴¹ Wasserstoff ist;
R³² eine Bindung zu Lₙ ist;
R⁸⁰ unabhängig ausgewählt ist aus:
-CO₂R⁸⁵, C₂-C₃-1-Alken, substituiert mit 0 bis 1 R⁸⁴, Aryl, substituiert mit 0 bis 1 R⁸⁴ und ungesättigtem Heterocyclus, substituiert mit 0 bis 1 R⁸⁴;
R⁸¹ H ist;
R⁸⁴ jeweils unabhängig ausgewählt ist aus: -CO₂R⁸⁵, -OR⁸⁵, -SO₃H, und -N(R⁸⁵)₂;
R⁸⁵ jeweils unabhängig ausgewählt ist aus: Wasserstoff und Methyl.

## Revendications

1. Produit radiopharmaceutique de la formule:
**[(Q)**_{**d'**}**L**_{**n**}**-C**_{**h'**}**]x-M**_{**t**}**(A**_{**L1**}**)**_{**y**}**(A**_{**L2**}**)**_{**Z**} (1)
et ses sels pharmaceutiquement acceptables, où
Q est une molécule biologiquement active sélectionnée dans le groupe: antagonistes de récepteur IIb/IIIa, ligands de récepteur IIb/IIIa, peptides liant la fibrine, peptides liant le leucocyte, peptides chimiotactiques, analogues de la somatostatine et peptides liant la sélectine;
d' est 1 à 3;
Lₙ est:
-(CR⁵⁵R⁵⁶)_{g}-[y¹(CR⁵⁵R⁵⁶)_{f}y²]_{f'}-(CR⁵⁵R⁵⁶)_{g}-,
où:
g" est 0-5;
f est 0-5;
f est 1-5;
y¹ et y², à chaque présence, sont indépendamment sélectionnés parmi:
O, NR⁵⁶, C=O, C(=O)O, OC(=O)O, C(=O)NH, C=NR⁵⁶, S, SO, SO₂, SO₃, NHC(=O), (NH)₂C(=O), (NH)₂C=S;
R⁵⁵ et R⁵⁶ sont indépendamment sélectionnés à chaque présence parmi:
hydrogène, alkyle et alkylaryle C₁-C₁₀;
x et y sont 1;
z est 1 ou 2;
Mₜ est ^{99m}Tc;
C_{h'} est sélectionné dans le groupe: R⁴⁰N=N⁺= et R⁴⁰R⁴¹N-N=; où
R⁴⁰ est indépendamment sélectionné à chaque présence dans le groupe: aryle substitué par 0-3 R⁵² et hétérocycle substitué par 0-3 R⁵²;
R⁴¹ est indépendamment sélectionné dans le groupe: hydrogène, aryle substitué par 0-1 R⁵², alkyle C₁-C₃ substitué par 0-1 R⁵² et un hétérocycle substitué par 0-1 R⁵²;
R⁵² est indépendamment sélectionné à chaque présence dans le groupe: une liaison à Lₙ, -CO₂R⁵³, -CH₂OR⁵³, -SO₃H, -SO₂R^{53a}, -N(R⁵³)₂, -N(R⁵³)₂, -N(R⁵³)₃+ -NHC(=NH)NHR⁵³, et -OCH₂CO₂H;
R⁵³ et R^{53a} sont chacun indépendamment sélectionnés à chaque présence dans le groupe: hydrogène et alkyle C₁-C₃;
A_{L1} est un aminocarboxylate fonctionnalisé;
A_{L2} est un ligand accessoire capable de stabiliser le produit radiopharmaceutique sélectionné dans le groupe: 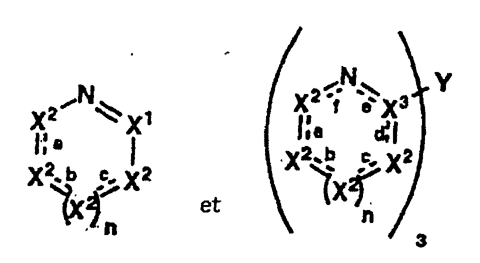 où:
n est 0
X¹ est indépendamment sélectionné à chaque présence dans le groupe: CR⁶⁴ et N;
X² est indépendamment sélectionné à chaque présence dans le groupe: CR⁶⁴, CR⁶⁴R⁶⁴, N, NR⁶⁴, O et S;
X³ est indépendamment sélectionné à chaque présence dans le groupe: C, CR⁶⁴ et N;
à condition que le nombre total de hétéroatomes dans chaque cycle du ligand A_{L2} soit 1 à 4;
Y est BR⁶⁴⁻;
et a, b, c, d, e et f indiquent les positions des doubles liaisons facultatives à condition que l'un de e et f soit une double liaison.
R⁶⁴ est indépendamment sélectionné à chaque présence dans le groupe:
H, alkyle C₁-C₃ substitué par 0-3 R⁶⁵, aryle substitué par 0-3 R⁶⁵ et R⁶⁵;
ou, bien alternativement, deux R⁶⁵ peuvent être pris ensemble avec l'atome ou les atomes auxquels ils sont attachés pour former un cycle aromatique ou hétérocyclique fusionné, substitué par 0-3 R⁶⁵;
R⁶⁵ est indépendamment sélectionné à chaque présence dans le groupe: -NO₂, -CO₂R⁶⁶, -OR⁶⁶, -SO₃H et -OCH₂CO₂H; et
R⁶⁶ est hydrogène.

2. Produit radiopharmaceutique de la revendication 1, où:
Q est une molécule biologiquement active sélectionnée dans le groupe: antagonistes de récepteur IIb/IIIa et peptides chimiotactiques;
d' est 1;
y¹ et y², à chaque présence, sont indépendamment sélectionnés parmi:
O, NR⁵⁶, C=O, C(=O)O, OC(=O)O, C(=O)NH, C=NR⁵⁶, NHC(=O), (NH)₂C(=O);
R⁵⁵ et R⁵⁶ sont hydrogènes;
z est 1;
R⁴⁰ est hétérocycle substitué par R⁵²;
R⁴¹ est hydrogène;
R⁵² est une liaison à Lₙ;
A_{L1} est la tricine;
X² est indépendamment sélectionné à chaque présence dans le groupe: CR⁶⁴, CR⁶⁴R⁶⁴, N, NR⁶⁴ et O;
X³ est N;
à condition que le nombre total de hétéroatomes dans chaque cycle du ligand A_{L2} soit 2 à 3;
R⁶⁴ est indépendamment sélectionné à chaque présence dans le groupe:
H, alkyle C₁-C₃ substitué par 0-1 R⁶⁵, aryle substitué par 0-1 R⁶⁵;
et R⁶⁵;
R⁶⁵ est indépendamment sélectionné à chaque présence dans le groupe: -NO₂, -CO₂R⁶⁶, -OR⁶⁶ et -SO₃H.

3. Produit radiopharmaceutique de la revendication 1, où:
Q est 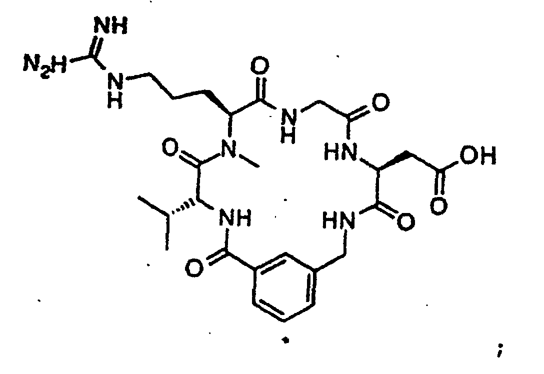
d' est 1;
Lₙ est attaché à Q à l'atome de carbone désigné par un * et a la formule:
-(C=O)NH(CH₂)₅C(=O)NH-; 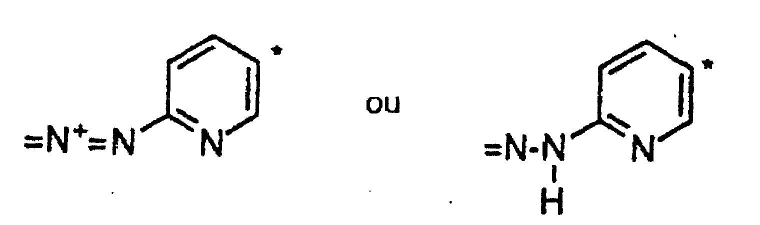
C_{h'} est , et est attaché à Lₙ à l'atome de carbone désigné par un *;
Mₜ est ^{99m}Tc;
A_{L1} est tricine;
x, y et z sont 1;
et A_{L2} est sélectionné dans le groupe: 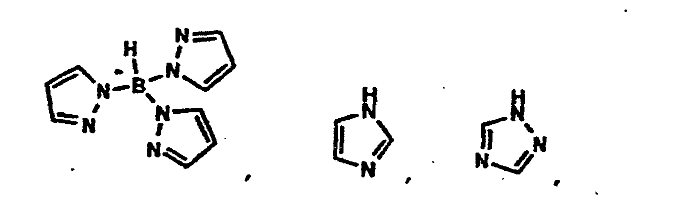 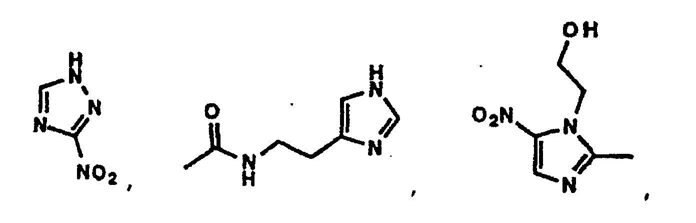 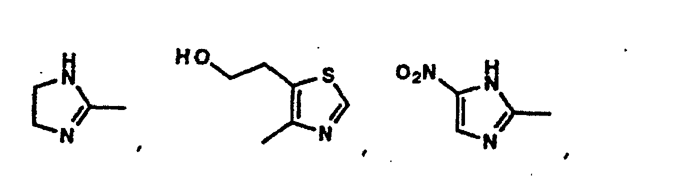 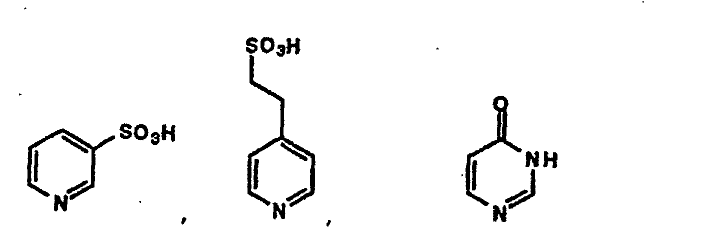 et

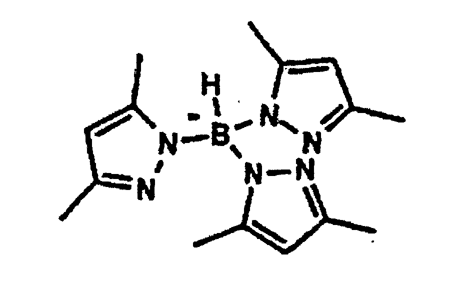

4. Composé selon l'une quelconque des revendications 1-3 pour utiliser en thérapie.

5. Utilisation d'un produit radiopharmaceutique selon l'une quelconque des revendications 1-3 pour la préparation d'un médicament à utiliser dans la radioimagerie d'un mammifère.

6. Utilisation d'un produit radiopharmaceutique selon l'une quelconque des revendications 1-3 pour la préparation d'un médicament à utiliser pour visualiser des sites d'un dépôt de plaquettes chez un mammifère par radioimagerie.

7. Utilisation d'un produit radiopharmaceutique selon l'une quelconque des revendications 1-3 pour la préparation d'un médicament à utiliser pour déterminer le dépôt de plaquettes chez un mammifère.

8. Utilisation d'un produit radiopharmaceutique selon l'une quelconque des revendications 1-3 pour la préparation d'un médicament à utiliser dans le diagnostic d'un trouble associé au dépôt de plaquettes chez un mammifère.

9. Kit pour la préparation d'un produit radiopharmaceutique, comprenant:
(a) une quantité prédéterminée d'un réactif stérile, pharmaceutiquement acceptable de la formule:
**(Q)**_{**d'**}**L**_{**n**}**-C**_{**h**}**;**
(b) une quantité prédéterminée d'un premier ligand accessoire stérile, pharmaceutiquement acceptable, A_{L1},
qui est un aminocarboxylate fonctionnalisé;
(c) une quantité prédéterminée d'un second ligand accessoire stérile, pharmaceutiquement acceptable, A_{L2}, sélectionné dans le groupe: 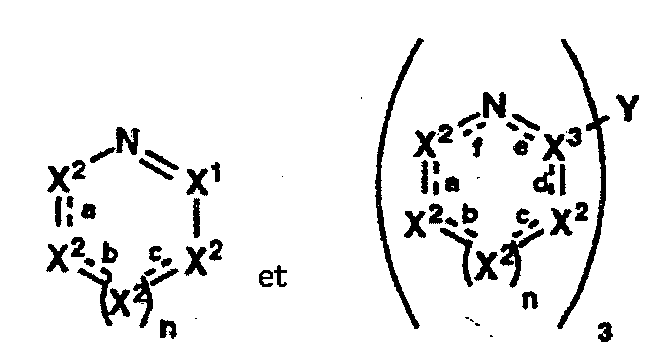
(d) une quantité prédéterminée d'un agent réducteur stérile, pharmaceutiquement acceptable;
et
(e) facultativement, une quantité prédéterminée d'un ou plusieurs composants stériles, pharmaceutiquement acceptables sélectionnés dans le groupe: ligands de transfert, tampons, auxiliaires de lyophilisation, auxiliaires de stabilisation, auxiliaires de solubilisation et bactériostats;
où:
Q, d', Lₙ, X¹, X², X³, n, Y, a, b, c, d, e et f sont tels que définis à la revendication 1;
Cₕ est un chélateur d'un métal d'un radionuclide sélectionné dans le groupe: R⁴⁰R⁴¹N-N=C(alkyle C₁-C₃)₂ et R⁴⁰NNH₂- et R⁴⁰R⁴¹N-N=CR⁸⁰R⁸¹, où,
R⁴⁰ est indépendamment sélectionné à chaque présence dans le groupe: une liaison à Lₙ, alkyle C₁-C₁₀ substitué par 0-3 R⁵², aryle substitué par 0-3 R⁵², cycloalkyle substitué par 0-3 R⁵², hétérocycle substitué par 0-3 R⁵², hétérocycloalkyle substitué par 0-3 R⁵², aralalkyle substitué par 0-3 R⁵² et alkaryle substitué par 0-3 R⁵²;
R⁴¹ est indépendamment sélectionné dans le groupe: hydrogène, aryle substitué par 0-3 R⁵², alkyle C₁-C₁₀ substitué par 0-3 R⁵² et un hétérocycle substitué par 0-3 R⁵²;
R⁵² est indépendamment sélectionné à chaque présence dans le groupe: une liaison à Lₙ, =O, F, C1, Br, I, -CF₃, -CN, -CO₂R⁵³, -C(=O)R⁵³, -C(=O)N(R⁵³)₂, -CHO, -CH₂OR⁵³, -OC(=O)R⁵³, -OC(=O)OR^{53a}, -OR⁵³, -OC(=O)N(R⁵³)₂, -NR⁵³C(=O)R⁵³, -NR⁵⁴C(=O)OR^{53a}, -N(R⁵³)₃+, -NR⁵³C(=O)N(R⁵³)₂, -NR⁵⁴SO₂N(R⁵³)₂, -NR⁵⁴SO₂R^{53a}, -SO₂N(R⁵³)₂, -N(R⁵³)₂, -NHC(=NH)NHR⁵³, -C(=NH)NHR⁵³, =NOR⁵³, NO₂, -C(=O)NHOR⁵³, -C(=O)NHNR⁵³R^{53a}, -OCH₂CO₂H, 2-(1-morpholino)éthoxy;
R⁵³, R^{53a} et R⁵⁴ sont chacun indépendamment sélectionnés à chaque présence dans le groupe: hydrogène, alkyle C₁-C₆ et une liaison à Lₙ;
R⁸⁰ et R⁸¹ sont indépendamment sélectionnés dans le groupe:
H, alkyle C₁-C₁₀, -CN, -CO₂R⁸⁵, -C(=O)R⁸⁵, -C(=O)N(R⁸⁵)₂, 1-alcène C₂-C₁₀ substitué par 0-3 R⁸⁴, 1-alkyne C₂-C₁₀ substitué par 0-3 R⁸⁴, aryle substitué par 0-3 R⁸⁴, hétérocycle insaturé substitué par 0-3 R⁸⁴ et carbocycle insaturé substitué par 0-3 R⁸⁴ à condition que quand l'un de R⁸⁰ et R⁸¹ est H ou alkyle, alors l'autre ne soit ni H ni alkyle;
ou, alternativement, R⁸⁰ et R⁸¹ peuvent être pris ensemble avec le radical de carbone divalent montré pour former: 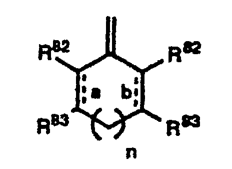 où:
R⁸² et R⁸³ peuvent être indépendamment sélectionnés dans le groupe:
H, R⁸⁴, alkyle C₁-C₁₀ substitué par 0-3 R⁸⁴, alcényle substitué par 0-3 R⁸⁴, alkynyle C₂-C₁₀ substitué par 0-3 R⁸⁴, aryle substitué par 0-3 R⁸⁴, hétérocycle substitué par 0-3 R⁸⁴ et carbocycle substitué par 0-3 R⁸⁴;
ou, alternativement, R⁸², R⁸³ peuvent être pris ensemble pour former un cycle aromatique ou hétérocyclique fusionné;
R⁸⁴ est indépendamment sélectionné à chaque présence dans le groupe: =O, F, C1, Br, I, -CF₃, -CN, -CO₂R⁸⁵, -C(=O)R⁸⁵, -C(=O)N(R⁸⁵)₂, -N(R⁸⁵)₃+, -CH₂OR⁸⁵, -OC(=O)R⁸⁵, -OC(=O)OR^{85a}, -OR⁸⁵, -OC(=O)N(R⁸⁵)₂, -NR⁸⁵C(=O)R⁸⁵, -NR⁸⁶C(=O)OR^{85a}, -NR⁸⁵C(=O)N(R⁸⁵)₂, -NR⁸⁶SO₂N(R⁸⁵)₂, -NR⁸⁶SO₂R^{85a}, -SO₃H, -SO₂R^{85a}, -SR⁸⁵, -S(=O)R^{85a}, -SO₂N(R⁸⁵)₂, -N(R⁸⁵)₂, -NHC(=NH)NHR⁸⁵, -C(=NH)NHR⁸⁵, =NOR⁸⁵, -C(=O)NHOR⁸⁵, -OCH₂CO₂H, 2-(1-morpholino)éthoxy; et
R⁸⁵, R^{85a} et R⁸⁶ sont chacun indépendamment sélectionnés à chaque présence dans le groupe: hydrogène et alkyle C₁-C₆.

10. Kit de la revendication 9, où:
A_{L1} est une tricine;
Q est une molécule biologiquement active sélectionnée dans le groupe: antagonistes de récepteur IIb/IIIa et peptides chimiotactiques;
d' est 1;
y¹ et y², à chaque présence, sont indépendamment sélectionnés parmi:
O, NR⁵⁶, C=O, C(=O)O, OC(=O)O, C(=O)NH, C=NR⁵⁶, NHC(=O), (NH)₂C(=O);
R⁵⁵ et R⁵⁶ sont hydrogènes;
R⁴⁰ est hétérocycle substitué par R⁵²;
R⁴¹ est hydrogène;
R⁵² est une liaison à Lₙ;
R⁸⁰ est indépendamment sélectionné à chaque présence dans le groupe: -CO₂R⁸⁵, 1-alcène C₂-C₃ substitué par 0-1 R⁸⁴, aryle substitué par 0-1 R⁸⁴ et hétérocycle insaturé substitué par 0-1 R⁸⁴;
R⁸¹ est H;
R⁸⁴ est indépendamment sélectionné à chaque présence dans le groupe: -CO₂R⁸⁵, -OR⁸⁵, -SO₃H et -N(R⁸⁵)₂;
R⁸⁵ est indépendamment sélectionné à chaque présence dans le groupe: hydrogène et méthyle;
X¹ est indépendamment sélectionné à chaque présence dans le groupe: CR⁶⁴ et N;
X² est indépendamment sélectionné à chaque présence dans le groupe: CR⁶⁴, CR⁶⁴R⁶⁴, N, NR⁶⁴ et O;
X³ est N;
Y est BR⁶⁴⁻;
à condition que le nombre total de hétéroatomes dans chaque cycle du ligand A_{L2} soit 2 à 3;
R⁶⁴ est indépendamment sélectionné à chaque présence dans le groupe:
H, alkyle C₁-C₃ substitué par 0-1 R⁶⁵, aryle substitué par 0-1 R⁶⁵ et R⁶⁵;
R⁶⁵ est indépendamment sélectionné à chaque présence dans le groupe: -NO₂, -CO₂R⁶⁶, -OR⁶⁶ et -SO₃H;
R⁶⁶ est hydrogène;
n est 0;
et a, b, c, d, e et f indiquent les positions des doubles liaisons facultatives à condition que l'un de e et f soit une double liaison.

11. Kit de la revendication 9, où:
A_{L2} est sélectionné dans le groupe: 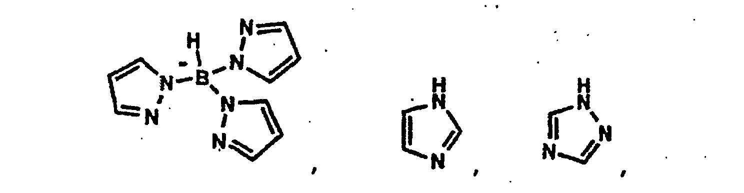 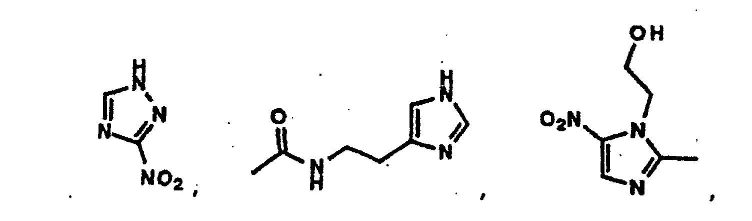 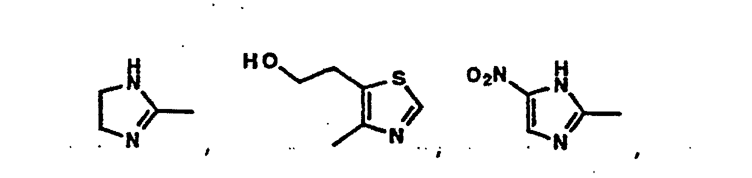 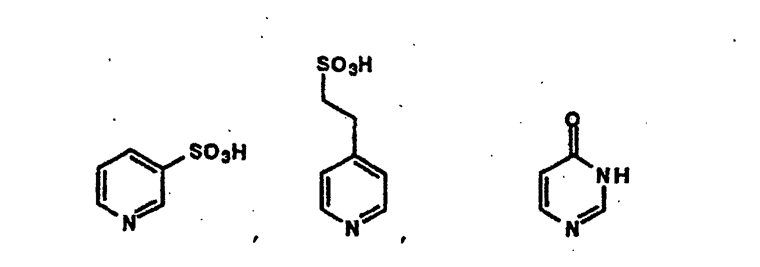 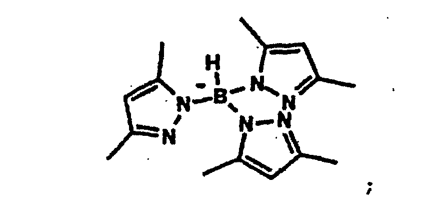
Q est 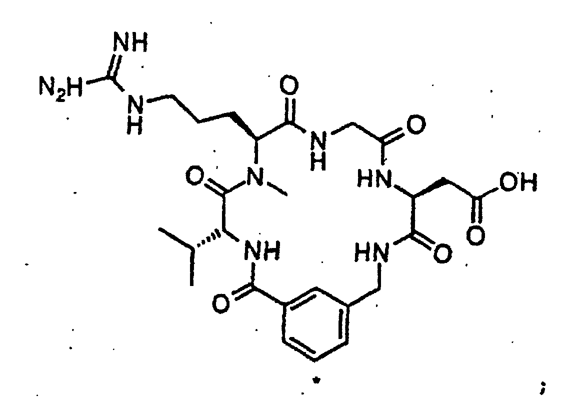
d' est 1;
Lₙ est attaché à Q à l'atome de carbone désigné par un * et a la formule:
-(C=O)NH(CH₂)5C(=O)NH-;
Cₕ est sélectionné parmi R⁴⁰NNH₂- et R⁴⁰R⁴¹N-N=CR⁸⁰R⁸¹, où,
R⁴⁰ est indépendamment sélectionné à chaque présence dans le groupe:
hétérocycle substitué par R⁵²;
R⁴¹ est hydrogène;
R⁵² est une liaison à Lₙ;
R⁸⁰ est indépendamment sélectionné à chaque présence dans le groupe: -CO₂R⁸⁵, 1-alcène C₂-C₃ substitué par 0-1 R⁸⁴, aryle substitué par 0-1 R⁸⁴ et hétérocycle insaturé substitué par 0-1 R⁸⁴;
R⁸¹ est H;
R⁸⁴ est indépendamment sélectionné à chaque présence dans le groupe: -CO₂R⁸⁵, -OR⁸⁵, -SO₃H et -N(R⁸⁵)₂;
R⁸⁵ est indépendamment sélectionné à chaque présence dans le groupe: hydrogène et méthyle.
